(19) **Europäisches Patentamt / European Patent Office / Office européen des brevets**

(11) **EP 4 311 565 B1**

(12) **EUROPEAN PATENT SPECIFICATION**

(45) Date of publication and mention
of the grant of the patent:
**26.11.2025 Bulletin 2025/48**

(21) Application number: **22186887.0**

(22) Date of filing: **26.07.2022**

(51) International Patent Classification (IPC):
*A61M 5/178* (2006.01)  *A61M 5/31* (2006.01)
*A61J 1/14* (2023.01)  *C03B 23/00* (2006.01)
*C03B 23/09* (2006.01)  *A61M 5/00* (2006.01)

(52) Cooperative Patent Classification (CPC):
**A61M 5/178; A61M 5/3135; A61M 5/3137;
C03B 23/092; C03B 23/094; C03B 23/097;**
A61M 5/008; A61M 2207/00

(54) **GLASS SYRINGE BARREL WITH INCREASED FLANGE BREAKING RESISTANCE**

GLASSPRITZENZYLINDER MIT ERHÖHTER FLANSCHBRUCHFESTIGKEIT

CYLINDRE DE SERINGUE EN VERRE PRÉSENTANT UNE MEILLEURE RÉSISTANCE À LA RUPTURE DE LA BRIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.01.2024 Bulletin 2024/05**

(73) Proprietor: **SCHOTT Pharma Schweiz AG
9000 St. Gallen (CH)**

(72) Inventors:
• **Oberhänsli, Roman
9000 St. Gallen (CH)**
• **Plapp, Michael
9322 Amriswil (CH)**
• **Lehner, Raphael
9053 Teufen (CH)**

(74) Representative: **Herzog IP Patentanwalts GmbH
Steinstraße 16-18
40212 Düsseldorf (DE)**

(56) References cited:
**US-A1- 2003 141 268  US-A1- 2018 296 762
US-A1- 2018 326 156  US-A1- 2019 125 978
US-A1- 2021 085 877**

**Description**

The present invention relates to a glass syringe barrel comprising with a top end through which a liquid can be ejected and a bottom end into which a plunger stopper can be pushed, the glass syringe barrel comprising in a direction from the top end to the bottom i) a cone region, ii) a shoulder region, iii) a body region and iv) a flange region, wherein the flange region is characterized by defined shape. The present invention also relates to a plurality of glass syringe barrels, to a process for the preparation of a glass syringe barrel, to a glass syringe barrel obtainable by such a process and to a syringe that comprises a glass syringe barrel.

In order to guarantee a reliable use of pharmaceutical products, pre-filled one-time-use syringes are available commercially. They permit a rapid injection of the product that they contain after a comparatively simple manipulation or handling. This sort of pre-filled syringe has a syringe barrel made from glass or polymer with a syringe head formed on it, in which either a syringe needle is integrated or which has a Luer connecting cone of a conical connection, if necessary, a lockable cone connection (Luer lock). On the side opposite the cone end (i. e., at the open end into which the plunger stopper can be introduced), the syringe body comprises a flange region. By allowing the operator's fingers to be positioned below the flange when the plunger is pushed in, the flange facilitates the injection of a drug located in the syringe when the plunger is pushed in, for example with the operator's thumb.

The aforementioned single-use syringe, also called a ready-made syringe, with a syringe barrel made of glass, is described in Norm DIN ISO 11040, in which, for example, the syringe barrel is described in part 4. The elastomeric standard piston stopper and standard piston rod made of polymer with a cruciform cross section are described in part 5.

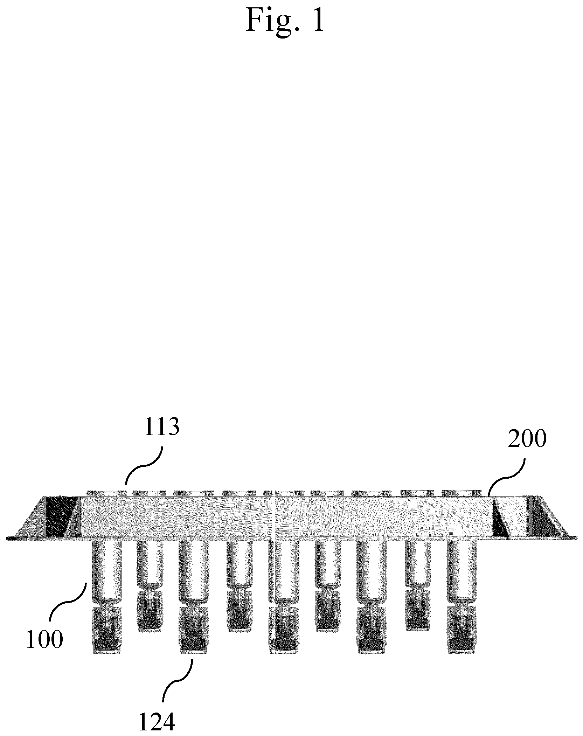

A known step in the preparation of pre-filled syringes is the manufacture of a syringe barrel and then the loading of a plurality of those syringe barrels into a so-called nest box, for storage and transport purposes to a location where the syringe barrels will be charged with a medicament and fitted with a plunger or a plug. The nest box comprises a nest tray with a base plate having a plurality of apertures arranged in an array and appropriately sized so that one syringe barrel is closely received in each aperture, with the flange region resting on the upper surface of a base plate. The syringe barrel is loaded into the tray with the lower side of the flange resting on the upper surface of the base plate (as shown in Fig. 1). Usually, the nest box is sealed with a cap and placed in a sterile bag.

However, with syringe bodies known from the prior art it has often been observed that, if they are inserted into the above-described nest box, they often do not rest level but partially canted with the lower side of the flange region resting on the base plate. This "canting" can be countered by forming the lower side of the flange region with as little "indentation" as possible, i.e., with a lower side of the flange region having an outer contour as even as possible. However, such a design of the lower side of the flange region is disadvantageous as it may lead to a reduction of the breaking resistance in the transition area between the flange region and the body region of the syringe body. Such a reduced breaking resistance may result in a breakage of the syringe body in the flange region if the syringes, particularly when being handled in automated filling and sealing machines, hit each other in the flange area.

US 2018/326156 A1 discloses a syringe comprising an elongate hollow body forming a reservoir intended to contain a substance to be injected, the body comprising a tip intended to receive a connection device, and an opening located at the opposite end of the body relative to the tip; a piston mounted slidably in the body and movable in translation along a longitudinal axis of the syringe between a retracted position, in which the piston is in abutment at the tip, and an extended position, in which the piston is in abutment at the opening; and gripping members provided on the body, and a gripping member provided at an opposite end of the piston relative to a head of the piston.

US 2019/125978 A1 discloses a needle protection assembly, adapted to protect a tip of a hypodermic needle, comprising a shield adapted, in a protected operative orientation of said needle protection assembly, to shield the tip of the hypodermic needle; a locking element including at least one slot, said slot including at least three surfaces corresponding to three operative orientations of said shield; said locking element having a base; at least one slot engaging element, functionally associated with said shield, said slot engaging element being disposed within said slot of said locking element and movable relative thereto, between said surfaces, so as to transition said shield between said three operative orientations; and at least one biasing element, adapted for axial biasing of said shield; said biasing element applying axial force between said base and said one slot engaging element; wherein said biasing element includes a torsion force adapted for relative rotation between said at least one slot engaging element and said locking element.

US 2018/296762 A1 discloses a method for filling an elongated cartridge having an opening in a proximal end thereof to an end of a cylindrical bore; the cartridge having a proximal portion; the cartridge including a lateral protrusion located distal to the proximal portion; the method comprising providing a syringe nest with a plurality of apertures therethrough arranged in a horizontal array; inserting said lateral protrusion and a distal portion of the cartridge through an aperture of the plurality of apertures while an axis of the cylindrical bore remains vertical, said lateral protrusion including an angled fluid path; hanging the cartridge from the syringe nest with said axis vertical and the distal portion of the cartridge and the lateral protrusion extending below said aperture and extending laterally past an edge of said cylindrical bore; and filling the cartridge with a drug product.

US 2003/141268 A1 discloses a nipple, comprising a fastening means including a sealing surface adapted to be fastened on a baby bottle, and a means forming a wall adapted to be contacted with a baby's mouth, said wall having at least one opening to allow milk to pass through, said wall means including a nipple portion with said opening being provided in said nipple portion, wherein said means forming said wall is comprised of polyisoprene rubber made with a neodymium catalyst.

US 2021/085877 A1 discloses a glass syringe barrel, comprising a top end through which a liquid can be ejected; a bottom end into which a plunger stopper can be pushed; a longitudinal axis through the top and bottom ends; and a cone region having a first end that corresponds to the top end and a second end, the cone region has a length l 1 and has an outer diameter d 1 at the second end; a shoulder region having a first end that is adjacent to the second end of the cone region and a second end; and a body region having a first end that is adjacent to the second end of the shoulder region and a second end that corresponds to the bottom end.

In general, it is an object of the present invention to at least partly overcome a disadvantage arising from the prior art. It is a further object of the present invention to provide a glass syringe barrel comprising a cone region, a shoulder region, a body region and a flange region, which, compared to similar glass syringe barrels known from the prior art, can be inserted into the apertures of a nest box as even as possible (i. e., without "canting"), but which still have a sufficiently high breaking resistance towards forces that are applied onto the flange region of the glass syringe barrel, making the syringe bodies particularly suitable to be handled in automated syringe fill-finish machines. It is a further object of the present invention to provide a glass syringe barrel comprising a cone region, a shoulder region, a body region and a flange region, wherein, compared to similar glass syringe barrels known from the prior art, less force is required to insert a plunger or stopper, also making the syringe barrels particularly suitable to be filled and sealed in automated syringe fill-finish machines. It is a further object of the present invention to provide a glass syringe barrel comprising a cone region, a shoulder region, a body region and a flange region, which, compared to similar glass syringe barrels known from the prior art, can more easily be washed by means of wash liquids that are introduced into the opening of a glass syringe body in an automatically operated cleaning station.

A contribution to at least partly solving at least one, preferably more than one, of the above objects is made by the independent claims. The dependent claims provide preferred embodiments which contribute to at least partly solving at least one of the objects.

A contribution to solving at least one of the objects according to the invention is made by a a glass syringe barrel 1 having a longitudinal axis $L_{barrel}$, the glass syringe barrel comprising a bottom end through which a liquid can be ejected and a top end into which a plunger stopper can be pushed, the glass syringe barrel comprising in a direction from the bottom end to the top end:

i) a cone region having a first end that corresponds to the bottom end of the glass syringe barrel and a second end;

ii) a shoulder region having a first end that is adjacent to the second end of the cone region and a second end;

iii) a body region with an outer diameter $d_1$ having a first end that is adjacent to the second end of the shoulder region and a second end;

iv) a flange region having at least in parts of the flange region a circular shape, the flange region having a first end that is adjacent to the second end of the body region and a second end that corresponds to the top end of the glass syringe barrel, wherein the flange region has an outer diameter d2 > d1 and wherein the flange region is characterized by an outer contour **c1** at its first end and an outer contour **c2** at its second end;

wherein, for any cut surface of the glass syringe barrel that includes the longitudinal axis $L_{bar-rel}$ and that is obtainable by cutting the glass syringe barrel in a plane at which the shape of the flange region is circular,

- $f_o$ and $f'_o$ are straight lines that run vertically to longitudinal axis $L_{barrel}$, wherein $f_o$ touches the outer contour **c1** at its deepest point $P_1$ and $f'_o$ touches the outer contour **c2** at its highest point $P_2$;

- $f(x)$ defines the absolute value of the vertical distance between any point of the outer contour **c1** and straight line $f_o$ at a position **x** (with $f(x) = 0$ at point $P_1$), wherein **x** is the horizontal distance between any given point on straight line $f_o$ and point $P_0$ at which straight line $f_o$ crosses a line $L_1$ that runs parallel to longitudinal axis $L_{barrel}$ and that touches the outer surface of the body region and wherein the maximum value for the term $f(x)$ in the range from x = $P_0$ to x = $P_1$ is $f(x)_{max}$ determined at position $x_{max}$, $f(x)_{max}$ representing the deepest indentation on the lower side of the flange region;

- wherein $f'(x)$ defines the absolute value of the vertical distance between any point of the outer contour **c2** and straight line $f'_o$ at a position **x** (with $f'(x) = 0$ at point $P_2$);

3

wherein the following condition is fulfilled:

$$f'(x_{max})/f(x)_{max} > 1;$$

preferably $f'(x_{max})/f(x)_{max} > 1.2$;
more preferably $f'(x_{max})/f(x)_{max} > 1.3$;
even more preferably $f'(x_{max})/f(x)_{max} > 1.4$;
most preferably $f'(x_{max})/f(x)_{max} > 1.5$;

wherein it is also preferred that preferably $f'(x_{max})/f(x)_{max}$ is less than 5, more preferably less than 3 and most preferably less than 2. Parameter $f(x)_{max}$ thus represents the deepest indentation on the lower side of the flange region and parameter $f'(x_{max})$ represents the indention on the upper side of the flange region determined at the same vertical position (i. e., at position $x = x_{max}$) as the deepest indentation on the lower side of the flange region.

In a preferred embodiment of the glass syringe barrel 1 according to the invention, $f'(x_{max})$ is in the range from 0.15 to 0.7 mm, preferably in the range from 0.15 to 0.5 mm, more preferably in the range from 0.2 to 0.45 mm and most preferably in the range from 0.2 to 0.4 mm.

In a further preferred embodiment of the glass syringe barrel 1 according to the invention, $f(x_{max})$ is in the range from 0.01 to 0.65 mm, preferably in the range from 0.1 to 0.4 mm, more preferably in the range from 0.15 to 0.3 mm and most preferably in the range from 0.2 to 0.3 mm.

In a further preferred embodiment of the glass syringe barrel 1 according to the invention, for any cut surface of the glass syringe barrel that includes the longitudinal axis $L_{barrel}$ and that is obtainable by cutting the glass syringe barrel in a plane at which the shape of the flange region is circular, D1 is the distance between point $P_1$ on the outer contour c1 and a point $P_3$ on outer contour c2, point $P_3$ being located vertically above point $P_1$ (= thickness of the flange region at its deepest point with $f(x) = 0$) and D2 is the distance between a point of the outer contour c1 and outer contour c2 at position $x_{max}$, wherein D1 and D2 are both determined in a direction that is parallel to longitudinal axis $L_{Barrel}$ and wherein the following condition is fulfilled:

$$D_1/D_2 < 1.6.$$

preferably $D_1/D_2 < 1.55$;
more preferably $D_1/D_2 < 1.50$;
even more preferably $D_1/D_2 < 1.47$;
most preferably $D_1/D_2 < 1.45$;

wherein it is also preferred that preferably $D_1/D_2$ is at least 1.2, more preferably at least 1.3 and most preferably at least 1.4.

In a further preferred embodiment of the glass syringe barrel 1 according to the invention, for any cut surface of the glass syringe barrel that includes the longitudinal axis $L_{barrel}$ and that is obtainable by cutting the glass syringe barrel in a plane at which the shape of the flange region is circular, D1 is in the range from 1.85 to 2.5 mm, preferably in the range from 1.88 to 2.4 mm, more preferably in the range from 1.91 to 2.3 mm, even more preferably in the range from 1.94 to 2.2 mm and most preferably in the range from 1.97 to 2.1 mm.

In a further preferred embodiment of the glass syringe barrel 1 according to the invention, for any cut surface of the glass syringe barrel that includes the longitudinal axis $L_{barrel}$ and that is obtainable by cutting the glass syringe barrel in a plane at which the shape of the flange region is circular, D2 is in the range from 1.1 to 2.0 mm, preferably in the range from 1.25 to 1.8 mm, more preferably in the range from 1.30 to 1.6 mm, even more preferably in the range from 1.35 to 1.55 mm and most preferably in the range from 1.4 to 1.5 mm.

In a further preferred embodiment of the glass syringe barrel 1 according to the invention, for any cut surface of the glass syringe barrel that includes the longitudinal axis $L_{barrel}$ and that is obtainable by cutting the glass syringe barrel in a plane at which the shape of the flange region is circular, d' is the distance between point $x'_{max}$ on the left side of the flange region and point $x''_{max}$ on the right side of the flange region on straight line $f_0$, $x'_{max}$ and $x''_{max}$ corresponding to the points at which function $f(x)$ reaches its maximum value on the left side and the right side of the flange region, respectively, wherein d' is in the range from 12.5 to 15 mm, preferably in the range from 12.5 to 14.5 mm, more preferably in the range from 12.75 to 14 mm and most preferably in the range from 13.0 to 13.5 mm.

In a further preferred embodiment of the glass syringe barrel 1 according to the invention, for any cut surface of the glass syringe barrel that includes the longitudinal axis $L_{barrel}$ and that is obtainable by cutting the glass syringe barrel in a plane at which the shape of the flange region is circular, d'' is the distance between point $P'_1$ on the left side of the flange region and point $P''_1$ on the right side of the flange region on straight line $f_0$, wherein d''/d2 is in the range from 0.8 to 0.95, preferably in the range from 0.82 to 0.9, more preferably in the range from 0.83 to 0.87 and most preferably in the range from 0.84 to 0.86.

In a further preferred embodiment of the glass syringe barrel 1 according to the invention, **d"** is in the range from 8 to 20 mm, preferably in the range from 9 to 19 mm, more preferably in the range from 10 to 18.5 mm and most preferably in the range from 11 to 18 mm and **$d_2$** is in the range from 12 to 22 mm, preferably in the range from 14 to 21 mm, more preferably in the range from 16 to 20 mm and most preferably in the range from 17 to 19 mm.

In a further preferred embodiment of the glass syringe barrel 1 according to the invention, for any cut surface of the glass syringe barrel that includes the longitudinal axis **$L_{barrel}$** and that is obtainable by cutting the glass syringe barrel in a plane at which the shape of the flange region is circular, a tangent that touches outer contour **c2** at position **$x_{max}$** includes an angle α with straight line **$f'_o$**, wherein α is in the range from 10 to 28 degree, preferably in the range from 14 to 26 degree, more preferably in the range from 18 to 25 degree and most preferably in the range from 20 to 24 degree.

In a further preferred embodiment of the glass syringe barrel 1 according to the invention, for any cut surface of the glass syringe barrel that includes the longitudinal axis **$L_{barrel}$** and that is obtainable by cutting the glass syringe barrel in a plane at which the shape of the flange region is circular,

- **$L_2$** is a straight line at touches the deepest point **$P'_1$** of the outer contour **c1** on the left side of the flange region and the deepest point **$P''_1$** of the outer contour **c1** on the right side of the flange region;

- **$L_3$** is a straight line at touches the outer contour **c1** on the left side of the flange region at a position x at which **f(x)** reaches the maximum value **$f(x)_{max}$** and the deepest point **$P''_1$** of the outer contour **c1** on the right side of the flange region;

wherein straight lines **$L_2$** and **$L_3$** enclose an angle β of less than 3 degree, preferably less than 2.5 degree, more preferably less than 2 degree, more preferably less than 1.5 degree and most preferably less than 1 degree.

In a further preferred embodiment of the glass syringe barrel 1 according to the invention, **$d_1$** is in the range from 6 to 20 mm, preferably in the range from 7.5 to 16 mm, more preferably in the range from 8 to 14 mm and most preferably in the range from 10 to 12 mm.

In a further preferred embodiment of the glass syringe barrel 1 according to the invention, difference **$d_2 - d_1$** is in the range from 5 to 8 mm, preferably in the range from 6 to 7.7 mm, more preferably in the range from 6.5 to 7.5 mm and most preferably in the range from 6.7 to 7.2 mm.

In a further preferred embodiment of the glass syringe barrel 1 according to the invention, the wall thickness **h** in the body region is in the range from 0.6 to 1.6 mm, preferably in the range from 0.7 to 1.5 mm, more preferably in the range from 0.8 to 1.4 mm, even more preferably in the range from 0.9 to 1.3 mm and most preferably in the range from 1 to 1.2 mm.

In a further preferred embodiment of the glass syringe barrel 1 according to the invention, the flange region, in a plane that is perpendicular to the longitudinal axis **$L_{barrel}$**, has the shape of a circle or the shape of a circle where two opposite sections of the circle have been removed.

In a further preferred embodiment of the glass syringe barrel 1 according to the invention, the outer surface of glass syringe barrel in the cone region is roughened or is provided with a coating.

In a further preferred embodiment of the glass syringe barrel 1 according to the invention, **$l_1$** is the length of the body region and wherein **$l_1$** is in the range from 30 to 60 mm, preferably in the range from 35 to 55 mm, more preferably in the range from 38 to 50 mm, even more preferably in the range from 39.5 to 45 mm and most preferably in the range from 39.5 to 41 mm.

In a further preferred embodiment of the glass syringe barrel 1 according to the invention, the glass syringe barrel has a nominal volume **V** and wherein **V** is in a range from 0.5 to 11 ml, preferably from 0.5 to 9 ml, more preferably from 0.5 to 7 ml, even more preferably from 0.5 to 5 ml, most preferably from 0.5 to 3 ml.

In a further preferred embodiment of the glass syringe barrel 1 according to the invention, the glass syringe barrel is rotation-symmetric around the longitudinal axis **$L_{barrel}$** that runs parallel to the body region and that preferably goes through the center of the top end and the bottom end.

In a further preferred embodiment of the glass syringe barrel 1 according to the invention, the glass syringe barrel is designed for a Luer-slip style connector and is sealed with a tip cap that is attached to the at least partially conically shaped upper portion.

In a further preferred embodiment of the glass syringe barrel 1 according to the invention, a needle is attached to the at least partially conically shaped upper portion via a Luer connector and wherein the needle is sealed with a needle cap.

In a further preferred embodiment of the glass syringe barrel 1 according to the invention, the glass is of a type selected from the group consisting of is selected from the group consisting of a borosilicate glass, an aluminosilicate glass, soda lime glass and fused silica.

In a further preferred embodiment of the glass syringe barrel 1 according to the invention, the glass syringe barrel comprises a coating that at least partially superimposes the interior surface of the body region.

A contribution to solving at least one of the objects according to the invention is made by a process for the production of a glass syringe barrel, more preferably a glass syringe barrel 1 according to the invention, comprising as process steps

I) providing a glass syringe barrel precursor having a longitudinal axis $L_{barrel}$, the glass syringe barrel precursor comprising a body region with a bottom end and a top end, the body region preferably having an outer diameter $d_1$;

II) heating the top end of the body region, while rotating the glass syringe barrel precursor around its longitudinal axis $L_{barrel}$, to prepare a ring of molten glass at the top end and forming a flange region by pushing the glass mass out beyond the edge of the body region at the top end of the glass syringe barrel precursor;

III) while the glass syringe barrel precursor is rotating around its longitudinal axis $L_{barrel}$, shaping the flange region obtained in process step II) to obtain a bottom side having an outer contour $c1$ and a top side having an outer contour $c2$, wherein the bottom side of the flange region is shaped by means of a shaping tool that comes into contact with the bottom side of the flange region;

wherein shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular,

- $f_o$ and $f'_o$ are straight lines that run vertically to longitudinal axis $L_{barrel}$, wherein $f_o$ touches the outer contour $c1$ at its deepest point $P_1$ and $f'_o$ touches the outer contour $c2$ at its highest point $P_2$;

- $f(x)$ defines the absolute value of the vertical distance between any point of the outer contour $c1$ and straight line $f_o$ at a position $x$ (with $f(x) = 0$ at point $P_1$), wherein x is the horizontal distance between any given point on straight line $f_o$ and point $P_0$ at which straight line $f_o$ crosses a line $L_1$ that runs parallel to longitudinal axis $L_{barrel}$ and that touches the outer surface of the body region and wherein the maximum value for the term $f(x)$ in the range from $x = P_0$ to $x = P_1$ is $f(x)_{max}$ determined at position $x_{max}$, $f(x)_{max}$ representing the deepest indentation on the lower side of the flange region;

- wherein $f'(x)$ defines the absolute value of the vertical distance between any point of the outer contour $c2$ and straight line $f'_o$ at a position $x$ (with $f'(x) = 0$ at point $P_2$);

wherein the following condition is fulfilled:

$$f'(x_{max})/f(x)_{max} > 1;$$

preferably $f'(x_{max})/f(x)_{max} > 1.2$;
more preferably $f'(x_{max})/f(x)_{max} > 1.3$;
even more preferably $f'(x_{max})/f(x)_{max} > 1.4$;
most preferably $f'(x_{max})/f(x)_{max} > 1.5$.

To form a flange region by pushing the glass mass out beyond the edge of the body region at the top end of the glass syringe barrel precursor in process step II), different approaches can be applied, which may also depend on the diameter and the thickness of the glass tube and thus also on the size of the glass syringe bodies to be prepared. According to one approach, the mass of molten glass can be pushed out beyond the edge of the body region at the top end of the glass syringe barrel precursor simply by means of the centrifugal force that is applied to the mass of molten glass while the glass syringe barrel precursor is rotating around its longitudinal axis $L_{barrel}$. According to a further approach, an air stream can be directed onto the mass of molten glass from the top to somehow push the mass of molten glass to the side. In this context it is preferred that this air stream is applied in an angle in a range between 20 to 70 degrees, preferably in the range from 40 to 60 degrees, relative to longitudinal axis $L_{barrel}$. Also, this air stream is preferably applied in an amount of 8 to 17 NL/min, more preferably in an amount of 10 to 14 NL/min.

In process step III) a moulding tool is moved directly to the bottom side of the flange region, preferably by means of a lifting cylinder. By means of this molding tool it is prevented that the flange region formed in process step II), which is still in a molten state, deforms downwards. The moulding tool thus stabilises the flange and ensures the flange height position. In this context it is preferred that the whole process of forming the final shape of the flange region does not require the use of any oil to actively lubricate the machine tools used to form the final shape of the flange region.

In a preferred embodiment of process 1 according to the invention in process step III) the flange region is brought into the desired flange shape by additionally directing an air stream to the top side of the flange region. The blown-on glass surface cools down faster and a sagging of the flange is thus prevented. In this context it is preferred that this air stream is applied in an angle in a range between 40 to 80 degrees, preferably in the range between 50 to 70 degrees, relative to longitudinal axis $L_{barrel}$. Also, the air stream is preferably applied in an amount of 4 to 10 NL/min, more preferably in an amount of 6 to 8 NL/min

In a further preferred embodiment of process 1 according to the invention, the shaping tool used to shape the bottom side of the flange region is made of carbon, preferably graphite R7710.

In a further preferred embodiment of process 1 according to the invention, in process steps II) and III) the glass syringe barrel precursor is rotating around its longitudinal axis $L_{barrel}$ with a rotating speed in the range from 700 to 900 rpm, preferably in the range from 740 to 790 rpm.

In a further preferred embodiment of process 1 according to the invention, shaping in process step III) is performed under such conditions that a flange region is obtained having at least in parts of the flange region a circular shape, the flange region having a first end that is adjacent to the second end of the body region and a second end that corresponds to the top end of the glass syringe barrel, wherein the flange region has an outer diameter $d_2 > d_1$ and wherein the flange region is characterized by an outer contour **c1** at its first end and an outer contour **c2** at its second end.

In a further preferred embodiment of process 1 according to the invention, shaping in process step III) is performed under such conditions that $f'(x_{max})/f(x)_{max}$ is less than 5, more preferably less than 3 and most preferably less than 2.

In a further preferred embodiment of process 1 according to the invention, shaping in process step III) is performed under such conditions that a flange region is obtained in which $f'(x_{max})$ is in the range from 0.15 to 0.7 mm, preferably in the range from 0.15 to 0.5 mm, more preferably in the range from 0.2 to 0.45 mm and most preferably in the range from 0.2 to 0.4 mm.

In a further preferred embodiment of process 1 according to the invention, shaping in process step III) is performed under such conditions that a flange region is obtained in which $f(x_{max})$ is in the range from .01 to 0.65 mm, preferably in the range from 0.1 to 0.4 mm, more preferably in the range from 0.15 to 0.3 mm and most preferably in the range from 0.2 to 0.3 mm.

In a further preferred embodiment of process 1 according to the invention, shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular, **D1** is the distance between point $P_1$ on the outer contour **c1** and a point $P_3$ on outer contour **c2**, point $P_3$ being located vertically above point $P_1$ (=thickness of the flange region at its deepest point) and **D2** is the distance between a point of the outer contour **c1** and outer contour **c2** at position $x_{max}$, wherein **D1** and **D2** are both determined in a direction that is parallel to longitudinal axis $L_{Barrel}$ and wherein the following condition is fulfilled:

$$\mathbf{D_1/D_2} < 1.6.$$

preferably $\mathbf{D_1/D_2} < 1.55$;
more preferably $\mathbf{D_1/D_2} < 1.50$;
even more preferably $\mathbf{D_1/D_2} < 1.47$;
most preferably $\mathbf{D_1/D_2} < 1.45$;

wherein it is also preferred that preferably $\mathbf{D_1/D_2}$ is at least 1.2, more preferably at least 1.3 and most preferably at least 1.4.

In a further preferred embodiment of process 1 according to the invention, shaping in process step III) is performed under such conditions that **D1** is in the range from 1.85 to 2.5 mm, preferably in the range from 1.88 to 2.4 mm, more preferably in the range from 1.91 to 2.3 mm, even more preferably in the range from 1.94 to 2.2 mm and most preferably in the range from 1.97 to 2.1 mm.

In a further preferred embodiment of process 1 according to the invention, shaping in process step III) is performed under such conditions that **D2** is in the range from 1.1 to 2.0 mm, preferably in the range from 1.25 to 1.8 mm, more preferably in the range from 1.30 to 1.6 mm, even more preferably in the range from 1.35 to 1.55 mm and most preferably in the range from 1.4 to 1.5 mm.

In a further preferred embodiment of process 1 according to the invention, shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular, **d'** is the distance between point $x'_{max}$ on the left side of the flange region and point $x''_{max}$ on the right side of the flange region on straight line $f_o$, $x'_{max}$ and $x''_{max}$ corresponding to the points at which function $f(x)$ reaches its maximum value on the left side and the right side of the flange region, respectively, wherein **d'** is in the range from 12.5 to 15 mm, preferably in the range from 12.5 to 14.5 mm, more preferably in the range from 12.75 to 14 mm and most preferably in the range from 13.0 to 13.5 mm.

In a further preferred embodiment of process 1 according to the invention, shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular, $d''$ is the distance between point $P'_1$ on the left side of the flange region and point $P''_1$ on the right side of the flange region on straight line $f_0$, wherein $d''/d2$ is in the range from 0.8 to 0.95, preferably in the range from 0.82 to 0.9, more preferably in the range from 0.83 to 0.87 and most preferably in the range from 0.84 to 0.86.

In a further preferred embodiment of process 1 according to the invention, shaping in process step III) is performed under such conditions that a flange region is obtained in which $d''$ is in the range from 8 to 20 mm, preferably in the range from 9 to 19 mm, more preferably in the range from 10 to 18.5 mm and most preferably in the range from 11 to 18 mm and $d_2$ is in the range from 12 to 22 mm, preferably in the range from 14 to 21 mm, more preferably in the range from 16 to 20 mm and most preferably in the range from 17 to 19 mm.

In a further preferred embodiment of process 1 according to the invention, shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular, a tangent that touches outer contour **c2** at position $x_{max}$ includes an angle $\alpha$ with straight line $f'_0$, wherein $\alpha$ is in the range from 14 to 26 degree, more preferably in the range from 18 to 25 degree and most preferably in the range from 20 to 24 degree.

In a further preferred embodiment of process 1 according to the invention, shaping in process step III) is performed under such conditions that a flange region is obtained in which, for any cut surface of the glass syringe barrel that includes the longitudinal axis $L_{barrel}$ and that is obtainable by cutting the glass syringe barrel in a plane at which the shape of the flange region is circular,

- $L_2$ is a straight line at touches the deepest point $P'_1$ of the outer contour **c1** on the left side of the flange region and the deepest point $P''_1$ of the outer contour **c1** on the right side of the flange region;

- $L_3$ is a straight line at touches the outer contour **c1** on the left side of the flange region at a position $x$ at which $f(x)$ reaches the maximum value $f(x)_{max}$ and the deepest point $P''_1$ of the outer contour **c1** on the right side of the flange region;

wherein straight lines $L_2$ and $L_3$ enclose an angle $\beta$ of less than 3 degree, preferably less than 2.5 degree, more preferably less than 2 degree, more preferably less than 1.5 degree and most preferably less than 1 degree.

In a further preferred embodiment of process 1 according to the invention, shaping in process step III) is performed under such conditions that a flange region with $d_1$ is in the range from 6 to 20 mm, preferably in the range from 7.5 to 16 mm, more preferably in the range from 8 to 14 mm and most preferably in the range from 10 to 12 mm.

In a further preferred embodiment of process 1 according to the invention, shaping in process step III) is performed under such conditions that a flange region is obtained in which $d_2 - d_1$ is in the range from 5 to 8 mm, preferably in the range from 6 to 7.7 mm, more preferably in the range from 6.5 to 7.5 mm and most preferably in the range from 6.7 to 7.2 mm.

In a further preferred embodiment of process 1 according to the invention, shaping in process step III) is performed by adjusting at least one of the following parameters:

a) the speed at which the glass syringe barrel precursor rotates around its longitudinal axis $L_{barrel}$;

b) the temperature of the glass in the ring of molten glass;

c) the pressure with which the gas stream that is directed onto the top of the ring of molten glass;

d) the position at which the shaping tool is located beneath the ring of molten glass.

Particularly preferred embodiments of process 1 according to the present invention are characterized in that the following parameters or combination of parameters are adjusted: a), b), c), d), a)b), a)c), a)d), b)c), b)d), c)d), a)b)c), a)b)d), b)c)d) and a)b)c)d), wherein the combination a)b)c)d) is most preferred.

In a further preferred embodiment of process 1 according to the present invention, the thickness h of the glass in the body region of the glass syringe barrel precursor is in the range from 0.6 to 1.6 mm, preferably in the range from 0.7 to 1.5 mm, more preferably in the range from 0.8 to 1.4 mm, even more preferably in the range from 0.9 to 1.3 mm and most preferably in the range from 1 to 1.2 mm.

In a further preferred embodiment of process 1 according to the present invention, the glass syringe barrel precursor provided in process step I) further comprises

- a cone region having a first end that corresponds to the bottom end of the glass syringe barrel precursor and a second end;

- a shoulder region having a first end that is adjacent to the second end of the cone region and a second end that corresponds to the bottom end of the body region.

In a further preferred embodiment of process 1 according to the present invention, the process further comprises the step of

IV) roughening the the outer surface of glass syringe barrel in the cone region or applying coating onto the outer surface of glass syringe barrel in the cone region.

In a further preferred embodiment of process 1 according to the present invention, the process further comprises the step of

V) cutting off two parts of the flange region that are located on opposing sides of the flange region, wherein cutting can be accomplished mechanically by means of a cutting device or can be accomplished thermally by means of a flame with which the glass is molten at the predetermined position and is then pulled apart.

In a further preferred embodiment of process 1 according to the present invention, the glass is of a type selected from the group consisting of is selected from the group consisting of a borosilicate glass, an aluminosilicate glass and fused silica.

In a further preferred embodiment of process 1 according to the present invention, the process further comprises the step of

VI) superimposing at least a part of the interior surface of the body region with a coating.

A contribution to solving at least one of the objects according to the invention is made by a glass syringe barrel 2 obtainable by the process of the invention according to the present invention, preferably by the process 1 according to any of its preferred embodiments. In a preferred embodiment of the glass syringe barrel 2, this glass syringe barrel 2 shows the technical features of the glass syringe barrel 1 of the invention.

A contribution to solving at least one of the objects according to the invention is made by a plurality of glass syringe barrels 1 or 2 according to the present invention, preferably a plurality of glass syringe barrels 1 according to any of its preferred embodiments, wherein at least one of the following conditions, preferably both of them, is/are fulfilled:

α) the relative standard deviation of the flange thickness **D1** is less than 6 %, preferably less than 5.5 % and more preferably less than 5 %;

β) the relative standard deviation of the length $l_2$ of the syringe is less than 12 %, preferably less than 11.5 % and more preferably less than 11 %.

According to a preferred embodiment of the plurality of glass syringe barrels 1 or 2 according to the present invention, at least one of the following conditions, preferably both of them, is/are fulfilled:

α) the relative standard deviation of the flange thickness **D1**

i) in case of a syringe having an outside diameter $d_1$ in the range from 7.5 to 8.5 mm, preferably in the range from 8 to 8.2 mm and a length $l_2$ in the range from 60 to 70 mm, preferably in the range from 65 to 67 mm, is less than 3 %, preferably less than 2.75 % and more preferably less than 2.5 %;

ii) in case of a syringe having an outside diameter $d_1$ in the range from 10 to 12 mm, preferably in the range from 10.5 to 11 mm and a length $l_2$ in the range from 45 to 55 mm, preferably in the range from 47 to 50 mm, is less than 3 %, preferably less than 2.75 % and more preferably less than 2.5 %;

iii) in case of a syringe having an outside diameter $d_1$ in the range from 10 to 12 mm, preferably in the range from 10.5 to 11 mm and a length $l_2$ in the range from 45 to 60 mm, preferably in the range from 52 to 54 mm, is less than 2.0 %, preferably less than 1.75 % and more preferably less than 1.5 %;

iv) in case of a syringe having an outside diameter $d_1$ in the range from 10 to 12 mm, preferably in the range from 10.5 to 11 mm and a length $l_2$ in the range from 50 to 60 mm, preferably in the range from 54 to 56 mm, is less than 2.5 %, preferably less than 2.25 % and more preferably less than 2.0 %;

v) in case of a syringe having an outside diameter $d_1$ in the range from 10 to 12 mm, preferably in the range from 10.5 to 11 mm and a length $l_2$ in the range from 60 to 75 mm, preferably in the range from 66 to 68 mm, is less than 5 %, preferably less than 4.75 % and more preferably less than 4.5 %;

vi) in case of a syringe having an outside diameter $d_1$ in the range from 10 to 12 mm, preferably in the range from 10.5 to 11 mm and a length $l_2$ in the range from 75 to 95 mm, preferably in the range from 84 to 86 mm, is less than 3 %, preferably less than 2.75 % and more preferably less than 2.5 %;

β) the relative standard deviation of the length $l_2$ of the syringe

i) in case of a syringe having an outside diameter $d_1$ in the range from 7.5 to 8.5 mm, preferably in the range from 8 to 8.2 mm and a length $l_2$ in the range from 60 to 70 mm, preferably in the range from 65 to 67 mm, is less than 10 %, preferably less than 9.5 % and more preferably less than 9 %;

ii) in case of a syringe having an outside diameter $d_1$ in the range from 10 to 12 mm, preferably in the range from 10.5 to 11 mm and a length $l_2$ in the range from 45 to 55 mm, preferably in the range from 47 to 50 mm, is less than 11.0 %, preferably less than 10.5 % and more preferably less than 10 %;

iii) in case of a syringe having an outside diameter $d_1$ in the range from 10 to 12 mm, preferably in the range from 10.5 to 11 mm and a length $l_2$ in the range from 45 to 60 mm, preferably in the range from 52 to 54 mm, is less than 10.5 %, preferably less than 10 % and more preferably less than 9.5 %;

iv) in case of a syringe having an outside diameter $d_1$ in the range from 10 to 12 mm, preferably in the range from 10.5 to 11 mm and a length $l_2$ in the range from 50 to 60 mm, preferably in the range from 54 to 56 mm, is less than 9.5 %, preferably less than 9 % and more preferably less than 8.5 %;

v) in case of a syringe having an outside diameter $d_1$ in the range from 10 to 12 mm, preferably in the range from 10.5 to 11 mm and a length $l_2$ in the range from 60 to 75 mm, preferably in the range from 66 to 68 mm, is less than 9.5 %, preferably less than 9 % and more preferably less than 8.5 %;

vi) in case of a syringe having an outside diameter $d_1$ in the range from 10 to 12 mm, preferably in the range from 10.5 to 11 mm and a length $l_2$ in the range from 75 to 95 mm, preferably in the range from 84 to 86 mm, is less than 11 %, preferably less than 10.5 % and more preferably less than 10 %;

wherein $l_2$ is the length of the upper most end on top of the flange region determined at position $P_2$ to the bottom end (i. e. the lower end of the conically shaped region).

The relative standard deviation of the of the maximum flange thickness **D1** and of length $l_2$ of the syringe in % is the standard deviation relative to the average value determined for these parameters in the plurality of syringe bodies.

"*A plurality of glass syringes*" in the sense of the present invention preferably comprises at least 10 glass syringes, preferably at least 25 glass syringes, more preferably at least 50 glass syringes, even more preferably at least 100 glass syringes even more preferably at least 200 glass syringes and most preferably at least 400 glass syringes. Furthermore, the plurality of glass syringes preferably has been collected arbitrarily and particularly has not been selected with regard to any property. For example, the plurality glass syringes may be the group of syringes which are packed together in a typical transport tray.

A contribution to solving at least one of the objects according to the invention is made by a syringe comprising

A) a glass syringe barrel 1 according to the present invention, preferably according to any of its preferred embodiments, a plurality 1 of glass syringe barrels according to the present invention, preferably according to any of its preferred embodiments, or the glass syringe barrel 2;

B) a plunger that has been pushed into the bottom end of the glass syringe barrel or into the bottom end of each of the glass syringe barrels contained in the plurality 1 of glass syringe barrels.
In a further preferred embodiment of the syringe according to the present invention, the syringe further comprises

C) a pharmaceutical composition that is filled into at least a part of the inner volume of the body region.

Glass syringe barrel

The glass syringe barrel according to the invention may have any size or shape which the skilled person deems appropriate in the context of the invention. The bottom end of the glass syringe barrel comprises an opening in the form of a channel located within the cone region through which a pharmaceutical composition that is contained in the glass syringe can be squeezed out of the glass syringe barrel and a top end with a flange region into which a plunger stopper can be pushed. Preferably, the glass syringe barrel is of a one-piece design that is prepared by providing a glass tube, preferably in form of a hollow cylinder, and forming the desired shape of the flange region. A preferred glass syringe barrel is a prefilled glass syringe barrel that is filled with a pharmaceutical preparation. Preferably, the glass syringe barrel is rotationally symmetrical around the longitudinal axis $L_{barrel}$ that preferably goes perpendicular through the centre of the body region.

Glass

The glass of the glass syringe barrel be any type of glass and may consist of any material or combination of materials which the skilled person deems suitable in the context of the invention. Preferably, the glass is suitable for pharmaceutical packaging. Particularly preferable, the glass is of type I, more preferably type I b, in accordance with the definitions of glass types in section 3.2.1 of the European Pharmacopoeia, 7th edition from 2011. Additionally, or alternatively preferable to the preceding, the glass is selected from the group consisting of a borosilicate glass, an aluminosilicate glass, soda lime glass and fused silica; or a combination of at least two thereof. For the use in this document, an aluminosilicate glass is a glass which has a content of $Al_2O_3$ of more than 8 wt.-%, preferably more than 9 wt.-%, particularly preferable in a range from 9 to 20 wt.-%, in each case based on the total weight of the glass. A preferred aluminosilicate glass has a content of $B_2O_3$ of less than 8 wt.-%, preferably at maximum 7 wt.-%, particularly preferably in a range from 0 to 7 wt.-%, in each case based on the total weight of the glass. For the use in this document, a borosilicate glass is a glass which has a content of $B_2O_3$ of at least 1 wt.-%, preferably at least 2 wt.-%, more preferably at least 3 wt.-%, more preferably at least 4 wt.-%, even more preferably at least 5 wt.-%, particularly preferable in a range from 5 to 15 wt.-%, in each case based on the total weight of the glass. A preferred borosilicate glass has a content of $Al_2O_3$ of less than 7.5 wt.-%, preferably less than 6.5 wt.-%, particularly preferably in a range from 0 to 5.5 wt.-%, in each case based on the total weight of the glass. In a further aspect, the borosilicate glass has a content of $Al_2O_3$ in a range from 3 to 7.5 wt.-%, preferably in a range from 4 to 6 wt.-%, in each case based on the total weight of the glass.

A glass which is further preferred according to the invention is essentially free from B. Therein, the wording "essentially free from B" refers to glasses which are free from B which has been added to the glass composition by purpose. This means that B may still be present as an impurity, but preferably at a proportion of not more than 0.1 wt.-%, more preferably not more than 0.05 wt.-%, in each case based on the weight of the glass.

Shape of the flange region

An important element of the glass syringe barrel 1 according to the invention is the shape of the flange region that is formed in process step III) of the process 1 according to the present invention, particularly the outer contour **c1** at the first end of the flange region and the outer contour **c2** at the second end of the flange region. The outer contour **c1** and the outer contour **c2** are characterized in that ratio of the depth of indention on the second end to the depth of indention on the first end $(f'(x_{max})/f(x)_{max})$ as determined at a position at which the indention on the end first end riches its maximum value is > 1. Surprisingly, it has been discovered that, if the outer contour **c1** at the first end of the flange region and the outer contour **c2** at the second end of the flange region are characterized by such a relative depth of indention, not only the positioning of the glass syringe barrels in the nest can be significantly improved by reducing the risk of the syringes "canting" and thus not coming to rest straight with the bottom side of the flange region on the base plate, the flange breaking resistance can also be significantly improved.

Pharmaceutical composition

In the context of the invention, every liquid pharmaceutical composition which the skilled person deems suitable to be used in a syringe comes into consideration. A pharmaceutical composition is a composition comprising at least one active ingredient. A preferred active ingredient is a vaccine. A further preferred pharmaceutical composition is a parenterialium, i.e. a composition which is intended to be administered via the parenteral route, which may be any route which is not enteral. Parenteral administration can be performed by injection, e.g. using a needle (usually a hypodermic needle) and a syringe.

MEASUREMENT METHODS

The following measurement methods are to be used in the context of the invention. Unless otherwise specified, the measurements have to be carried out at an ambient temperature of 23°C, an ambient air pressure of 100 kPa (0.986 atm) and a relative atmospheric humidity of 50%.

### Determination **of f(x)** and **f'(x)**

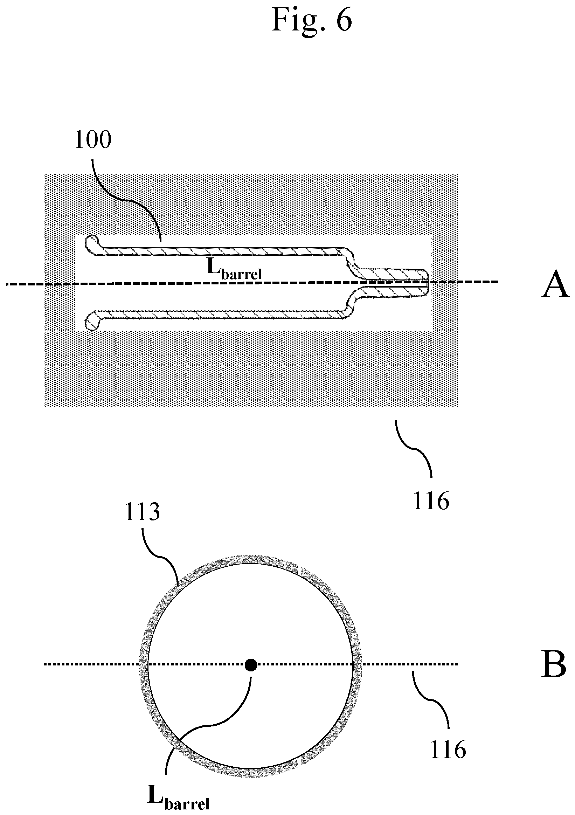

- The outer contour **c1** and **c2** of the glass syringe barrel in the flange region defined by the function **f(x)** and **f'(x),** respectively, can be determined in a non-destructive manner using a profile projector. This approach is particularly suitable for glass syringe barrels that have been chemically and/or thermally tempered and that therefore cannot be easily sliced in half without the glass cracking or bursting. For determining the values for **f(x)** and **f'(x)** in a non-destructive manner the outer contour **c1** and **c2** of the glass syringe barrel in the flange region is visualized using a Mitutoyo PJ-3000 profile projector. The profile projector has a 10X magnification and is operated with transmitted light illumination. The barrels are placed in Hallbrite® BHB (a butyloctyl salicylate obtainable from the Hallstar Company, Chicago, USA), which is filled into a glass bowl. Hallbrite® BHB is used to visualize the outer contour of the flange region. It is ensured that the cross-section of the glass syringe barrel that is inspected in the profile projector corresponds to the plane that is centrically located in the glass syringe barrel and that comprises the longitudinal axis $L_{barrel}$ of the glass syringe barrel, i. e. the axis that goes perpendicular through the barrel (see Figures 6A and 6B).
- To improve the measuring accuracy, the outer contour **c1** and **c2** of the glass syringe barrel in the flange region can also be determined from a physical cross-sectional cut parallel along to the longitudinal axis $L_{barrel}$ of the barrel (it is again ensured that the cross-section of the glass syringe barrel corresponds to the plane that is centrically located in the glass syringe barrel and that comprises the longitudinal axis of the glass syringe barrel as shown in Figures 6A and 6B). For preparation without breakage, the barrel may be embedded into transparent 2-component epoxy resin, for example STRUERS GmbH, EpoFix Resin, or other suitable materials. After curing of the epoxy resin, a cross-sectional cut parallel along to the barrel axis can be achieved by machine-supported sawing, grinding and polishing. Geometrical features of the barrel can then be determined (measured) by means of non-distorting image capturing and geometrical analysis software tools.

The relevant outer contour **c1** and **c2** of the glass syringe barrel in the flange region can be extracted and numerically approximated from the images obtained by means of the two approaches described above. For the extraction of the relevant contour, the images undergo the image processing steps implemented in Python [https://www.python.org/] based on the image processing library OpenCV [https://open-cv.org/].

First, the images are denoised using a median filter. The denoised images are then processed with an edge detection algorithm based on a Sobel filter, in which the contours are identified by thresholding the gradient image.

### Determination of the flange thickness **D1**

The flange thickness **D1** is determined by means of a Round Flange Camera.

### EXAMPLE

A glass tube (FIOLAX® clear, SCHOTT AG, Germany) having an outer diameter $d_1$ of 10.85 mm and a wall thickness **n** of 1.1 mm made of borosilicate glass is loaded into the head of a rotary machine. While rotating around its longitudinal axis $L_{tube}$ one end of the glass tube (i. e. at the end of which the Luer cone will be localized) is heated to its softening point with flames (see Fig. 11A). While the glass tube is rotating around its longitudinal axis the end that has been heated is shaped using molding tools that act on predetermined positions of the outer surface of the glass tube at the first end to form the cone region. In a further process step the glass tube, while rotating around its longitudinal, is cut at a predetermined position above the first end to obtain a glass tube comprising a first end that is cone shaped and second end. The glass tube of the thus obtained glass syringe barrel precursor is then heated at the second end, while rotating around its longitudinal, to a temperature above its glass transition temperature with a flame. While the glass tube is still rotating around its longitudinal at a rotational speed of 750 rpm, the mass of molten glass is pushed out beyond the edge of the body region at the top end of the glass syringe barrel precursor simply by means of the centrifugal force, thereby forming a flange region.

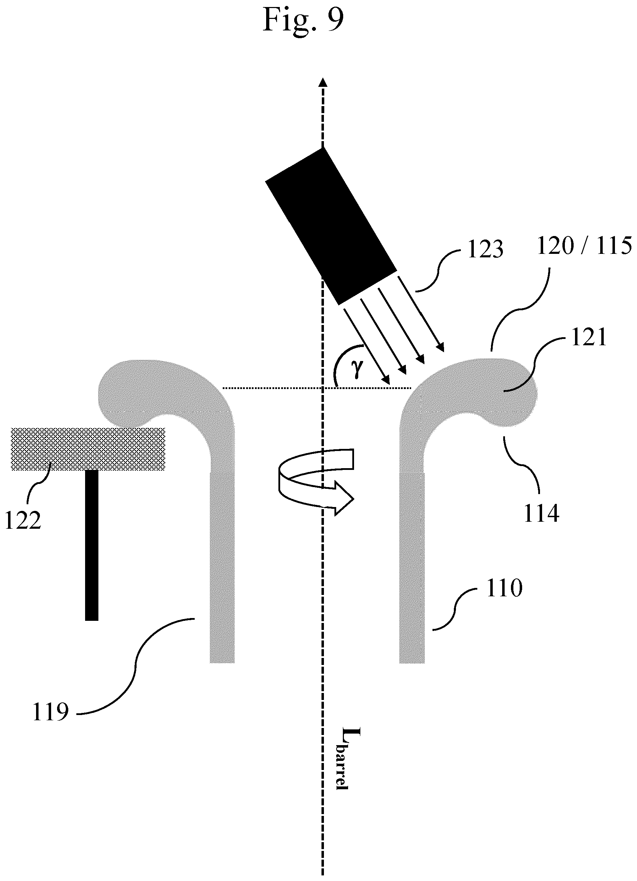

In a further process step, the final shape of the flange region is obtained by means of the set up shown in Fig. 9. As shown in that figure, the final shape on the bottom side of the flange region is prepared by bringing the mass of molten glass into contact with a shaping tool in such a way that the shaping tool somehow lifts the mass of glass upwards, thereby reducing the deepest indentation on the lower side of the flange region **(f(x)$_{max}$).** At the same time, the top side of the flange region is cooled by means of an air stream (applied in an angle of 50°) that primarily serves to cool down the surface on the top side of the flange region, thereby stabilizing the shape of the flange region on that side. In a Comparative Example, process step III) (i. e., the treatment with the shaping tool and the air stream) has been omitted.

|  | Comparative Example 1 | Example 1 |
|---|---|---|
| $f'(x_{max})/f(x)_{max}$ | 0.08 | 1.33 |
| $f'(x)_{max}$ [mm] | 0.06 | 0.32 |
| $f(x)_{max}$ [mm] | 0.77 | 0.24 |
| $\alpha$ [degree] | 8 | 22 |
| $\beta$ [degree] | 3.17 | 0.97 |
| $D_1$ [mm] | 1.81 | 1.99 |
| $D_2$ [mm] | 1.05 | 1.42 |
| d' [mm] | 12.29 | 13.27 |
| d" [mm] | 15.67 | 15.23 |
| $d_2$ [mm] | 17.55 | 17.88 |

It has been observed that the glass syringe barrels according to the present invention do rest more level when being placed in a nest box as (i. e., they are less prone to "canting"), compared to the glass syringe barrels known from the prior art.

Unless otherwise specified in the description or the particular Figure:

Figure 1 shows a cross-sectional view of a nest 200 holding glass syringe barrels 100;

Figure 2 shows a cross-sectional enlarged view a syringe barrel 100 according to the present invention;

Figure 3 shows a cross-sectional enlarged view of a part of the flange region 113 in a glass syringe barrel 100 and the determination of the outer contour **c1** and **c2** by means of functions **f(x)** and **f'(x)**;

Figure 4 shows in a further a cross-sectional enlarged view of the shape of a flange region 113 in a glass syringe barrel 100 according to the present invention and the determination of $\beta$;

Figure 5 shows in a further a cross-sectional enlarged view of the shape of a flange region 113 in a glass syringe barrel 100 according to the present invention and the determination of **d'** and **d"**;

Figur 6A shows in a side view the localization of plane 116 that is used to determine the outer contour **c1** and **c2** in the flange region 113;

Figure 6B shows in a top view the localization of plane 116 that is used to determine the outer contour **c1** and **c2** in the flange region 113;

Figures 7A,B show a top-view two different shapes of the flange region;

Figure 8 shows a cross-sectional enlarged view of a part a syringe according to the present invention into which a plunger 118 has been inserted into the opening at the side of the flange region 113;

Figure 9 illustrates step III) of process 1 according to the invention for the preparation of a glass syringe barrel 100.

Figure 1 shows a cross-sectional view of a nest 200 that is holding a plurality of glass syringe barrels 100. Such a nest 200 is used in automated syringe fill-finish machines, in which a plurality of syringe barrels 100 that are closed at the conus-shaped end by means of appropriate closure systems 124 are filled with a pharmaceutical composition. The nest 200 comprises a plurality of recesses (not visible in Fig. 1) into which the syringe barrels 100 are introduced in such a way that they rest with the lower (first) end 114 on the upper surface of the nest 200.

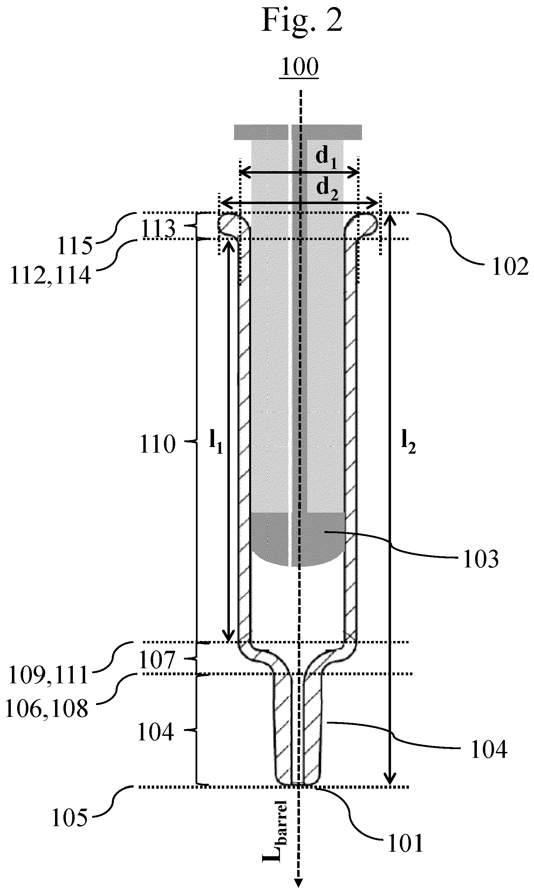

Fig. 2 shows a cross-sectional enlarged view a syringe barrel 100 according to the present invention. The syringe barrel 100 has a longitudinal axis $\mathbf{L_{barrel}}$ and comprises a bottom end 101 through which a liquid can be ejected and which is usually shaped in the form of a conus, and a top end 102 into which a plunger stopper 103 (or a plug) can be pushed. The glass syringe barrel 100 comprises in a direction from the bottom end 101 to the top end 102 a cone region 104 having a first end 105 that corresponds to the bottom end 101 of the glass syringe barrel 100 and a second end 106, a shoulder region 107 having a first end 108 that is adjacent to the second end 106 of the cone region 104 and a second end 109, a body region 110 with an outer diameter $\mathbf{d_1}$ having a first end 111 that is adjacent to the second end 109 of the shoulder region 107 and a second end 112, and a flange region 113 which, at least in parts of the flange region 113, is formed in the shape of a plate, the flange region 113 having a first end 114 that is adjacent to the second end 112 of the body region 110 and a second end 115 that corresponds to the top end 102 of the glass syringe barrel 100, wherein the flange region 113 has an outer diameter $\mathbf{d_2} > \mathbf{d_1}$. As also shown in Fig. 2, the length of the body region 110 is $\mathbf{l_1}$ and the length of the upper most end on top of the flange region 113 determined at position $\mathbf{P_2}$ to the bottom end (i. e. the lower end of the conically shaped region) is $\mathbf{l_2}$.

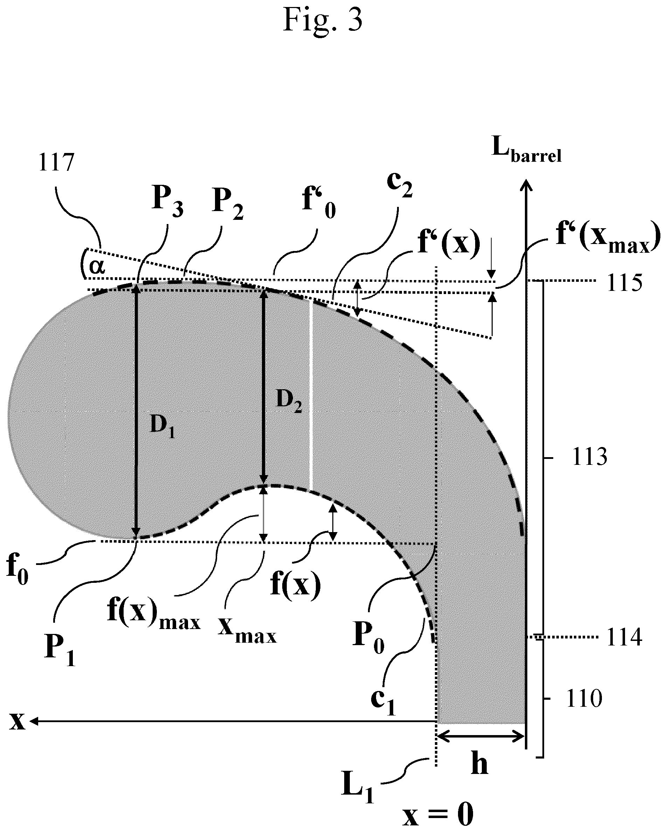

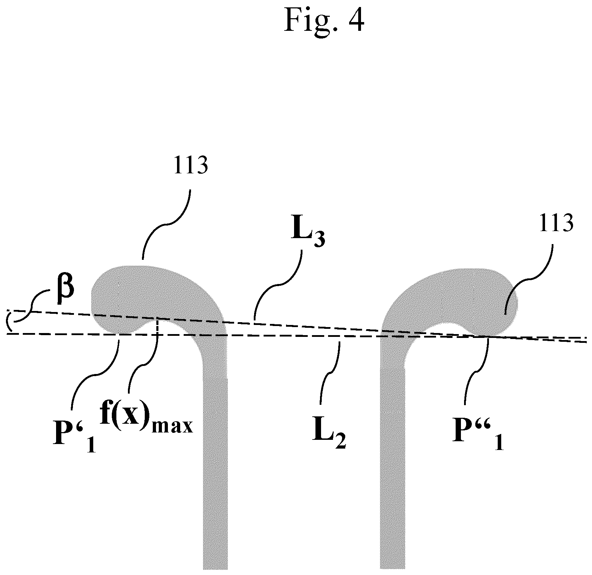

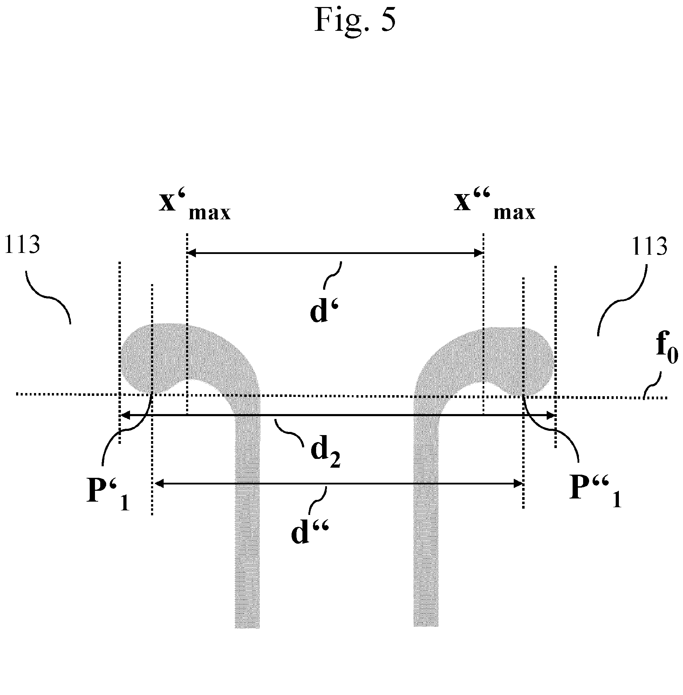

Fig. 3 shows a cross-sectional enlarged view of a part of the flange region 113 in a glass syringe barrel 100 and the determination of the outer contour **c1** and **c2** by means of functions **f(x)** and **f'(x)**. Figure 3 just shows the left part of the flange region in a cross-section of the glass syringe barrel 100 that is located in a plane 116 being centrically located in the glass syringe barrel 100 and comprising the longitudinal axis $L_{barrel}$ of the glass syringe barrel (see Fig. 6A and 6B). The corresponding part on the right (which is shown in Figures 4 and 5) is not shown in Fig. 3. As can be seen in Fig. 3, flange region 113 has an outer diameter $d_2 > d_1$ and is characterized by an outer contour **c1** at its first end 114 (dotted lined on the top surface of the flange region 113) and an outer contour **c2** at its second end 115 (dashed line on the bottom of the flange region 113). $f_o$ and $f'_o$ are straight lines that run vertically to longitudinal axis $L_{barrel}$, wherein $f_o$ touches the outer contour **c1** at its deepest point $P_1$ and $f'_o$ touches the outer contour **c2** at its highest point $P_2$. As can also be seen in Fig. 3, **f(x)** defines the absolute value of the vertical distance between any point of the outer contour **c1** and straight line $f_o$ at a position **x** (with **f(x)** = 0 at point $P_1$), wherein x is the horizontal distance between any given point on straight line $f_o$ and point $P_0$ at which straight line $f_o$ crosses a line $L_1$ that runs parallel to longitudinal axis $L_{barrel}$ and that touches the outer surface of the body region 110. The maximum value for the term **f(x)** in the range from $x = P_0$ to $x = P_1$ is $f(x)_{max}$ determined at position $x_{max}$ **f'(x)** defines the absolute value of the vertical distance between any point of the outer contour **c2** and straight line $f'_o$ at a position **x** (with **f'(x)** = 0 at point $P_2$), The glass syringe barrel according to the present invention is characterized in that the flange region 113 has a shape such that condition $f'(x_{max})/f(x)_{max} > 1$ is fulfilled. As can also be seen in Fig. 3, **D1** is the distance between point $P_1$ on the outer contour **c1** and a point $P_3$ on outer contour **c2,** point $P_3$ being located vertically above point $P_1$ (=thickness of the flange region at its deepest point). According to a preferred embodiment of the glass syringe barrel according to the present invention condition $D_1/D_2 < 1.5$ is fulfilled.

Fig. 4 shows in a further a cross-sectional enlarged view of the shape of a flange region 113 in a glass syringe barrel 100 according to the present invention and the determination of β. As shown in Fig. 4, $L_2$ is a straight line at touches the deepest point $P'_1$ of the outer contour c1 on the left side of the flange region 113 (that is also shown in Fig. 3) and the deepest point $P''_1$ of the outer contour **c1** on the right side of the flange region 113 (that is not shown in Fig. 3). $L_3$ is a straight line at touches the outer contour **c1** on the left side of the flange region 113 at a position **x** at which **f(x)** reaches the maximum value $f(x)_{max}$ and the deepest point $P''_1$ of the outer contour **c1** on the right side of the flange region 113. According to a preferred embodiment of the glass syringe barrel according to the present invention condition straight lines $L_2$ and $L_3$ enclose an angle β of less than 2.5 degree.

Fig. 5 shows in a further a cross-sectional enlarged view of the shape of a flange region 113 in a glass syringe barrel 100 according to the present invention and the determination of d' and **d''**. As shown in Fig. 5, **d'** is the distance between point $x'_{max}$ on the left side of the flange region 113 and point $x''_{max}$ on the right side of the flange region 113 on straight line $f_o$, $x'_{max}$ and $x''_{max}$ corresponding to the points at which function **f(x)** reaches its maximum value on the left side and the right side of the flange region 113, respectively. According to a preferred embodiment of the glass syringe barrel according to the present invention d' is in the range from 9 to 14 mm. As can also be seen in Fig. 5, **d''** is the distance between point $P'_1$ on the left side of the flange region 113 and point $P''_1$ on the right side of the flange region 113 on straight line $f_0$. According to a further preferred embodiment of the glass syringe barrel according to the present invention wherein **d''** is preferably in the range from 8 to 20 mm. According to a further preferred embodiment of the glass syringe barrel according to the present invention, $d''/d_2$ is in the range from 0.8 to 0.95.

Figures 6A and 6B show in a side view and in a top view the localization of plane 116 in the glass syringe barrel 100 that is used to determine the outer contour c1 and c2 in the flange region 113. Plane 116 corresponds to the plane that is centrically located in the glass syringe barrel 100 and that comprises the longitudinal axis $L_{barrel}$ of the glass syringe barrel 100.

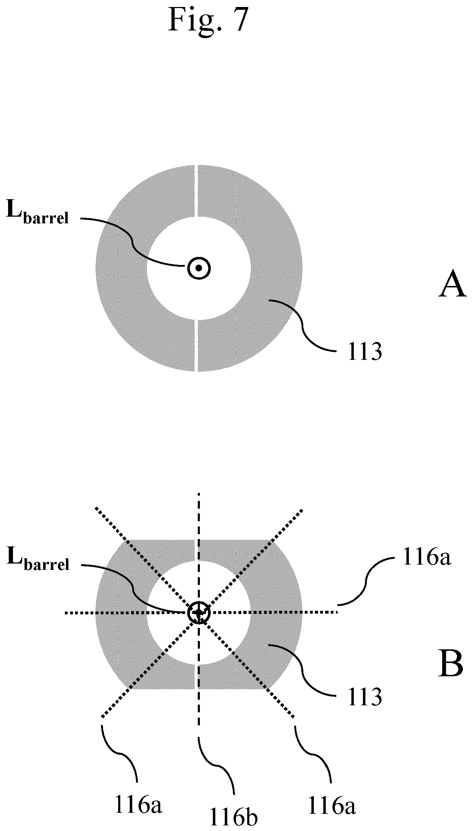

Fig. 7A and 7B show a top-view two different shapes of the flange region 113. The flange region 113 shown in Fig. A as a (full) circular shape, whereas the flange region 113 shown in Fig. 7B has been derived from a flange region 113 by cutting off pieces of the flange region 113 on two opposite sides. In case of such "cut-flange", plane 116 that is used to determine the outer contour **c1** and **c2** in the flange region 113 as shown in Fig. 6A and 6B is located in a plane being centrically located in the glass syringe barrel 100 at a position at which the outer contour of the flange region 113 is circular shaped. Planes 116 that fulfil this requirement are the dotted lines 116a, whereas a plane that does not fulfil this requirement is dashed line 116b.

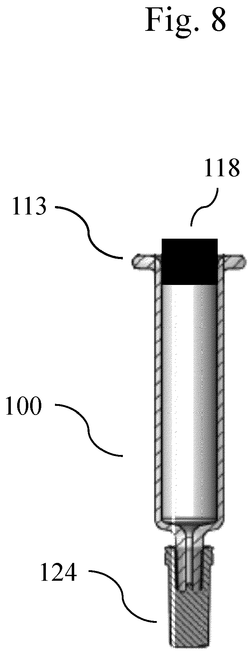

Figure 8 shows a glass syringe barrel 100 according to the present invention with a tip cap 124 attached to the conically shaped side at the lower end and with a plug 118 that is inserted into the open end of the glass syringe barrel 100 at the upper end at which the flange region 113 is located.

Fig. 9 illustrates step III) of process 1 according to the invention for the preparation of a glass syringe barrel 100. In the process for the preparation of a glass syringe barrel 100 according to the present invention in a first process step a glass syringe barrel precursor 119 is provided having a longitudinal axis $L_{barrel}$, the glass syringe barrel precursor 119 comprising a body region 110 with a bottom end and a top end, the body region 100 preferably having an outer diameter $d_1$. In a particular embodiment of the process according to the present invention, the bottom end is already shaped in the form of a conus to obtain the cone region 104 as shown in Fig. 2. In a further process step the top end 120 of the body region 110, while rotating the glass syringe barrel precursor 119 around its longitudinal axis $L_{barrel}$, is heated to prepare a ring of molten glass 121 at the top end, wherein heating is preferably accomplished by means of flames from a gas burner that are directed onto the top surface 115 of the glass syringe barrel precursor 119 to obtain a molten ring of glass 121. A flange region 113 is formed by pushing the glass mass 121 out beyond the edge of the body region 110 at the top end of the glass syringe barrel precursor 119. In process step III) of the process according to the present invention (shown in Fig. 9), the thus obtained flange region 113 is finally shaped to obtain a bottom side 114 having an outer contour **c1** and a top side 115 having an outer contour **c2**. As shown in Fig. 9, the final shape on the bottom side 114 of the flange region 113 is prepared by bringing the mass of molten glass 121 into contact with a shaping tool 122 in such a way that the shaping tool 122 somehow lifts the mass of glass 121 upwards, thereby reducing the deepest indentation on the lower side of the flange region 113 **(f(x)$_{max}$)**. At the same time, the top side 115 of the flange region 113 is cooled by means of an air stream 123 (applied in an angle of approximately 50°) that primarily serves to cool down the surface of the flange region 113 on the top side 115, thereby stabilizing the shape of the flange region 113 on that side.

LIST OF REFERENCE NUMERALS

| | |
|---|---|
| **100** | glass syringe barrel according to the invention |
| **101** | bottom end |
| **102** | top end |
| **103** | plunger stopper |
| **104** | cone region |
| **105** | first end of the cone region 104 (= bottom end 101) |
| **106** | second end of the cone region 104 (= first end 108 of the shoulder region 107) |
| **107** | shoulder region |
| **108** | first end of the shoulder region 107 (= second end 106 of the cone region 104) |
| **109** | second end of the shoulder region 107 (= first end 111 of the body region 110) |
| **110** | body region |
| **111** | first end of the body region 110 (= second end 109 of the shoulder region 107) |
| **112** | second end of the body region 110 (= first end 114 of the flange region 113) |
| **113** | flange region |
| **114** | first end (bottom side) of the flange region 113 (= the second end 112 of the body region 110) |
| **115** | second end (top side) of the flange region 113 (= top end 102) |
| **116** | cross-sectional plane in the middle of the glass syringe barrel 100 |
| **117** | a tangent that touches outer contour **c2** at position $x_{max}$ |
| **118** | plug |
| **119** | glass syringe barrel precursor |
| **120** | top end of glass syringe barrel precursor 119 |
| **121** | ring of molten glass |
| **122** | shaping tool |
| **123** | gas stream |
| **124** | tip cap |

**Claims**

1.  A glass syringe barrel (100) having a longitudinal axis **L$_{barrel}$**, the glass syringe barrel (100) comprising a bottom end (101) through which a liquid can be ejected and a top end (102) into which a plunger stopper (103) can be pushed, the glass syringe barrel (100) comprising in a direction from the bottom end (101) to the top end (102):

    i) a cone region (104) having a first end (105) that corresponds to the bottom end (101) of the glass syringe barrel (100) and a second end (106);
    ii) a shoulder region (107) having a first end (108) that is adjacent to the second end (106) of the cone region (104) and a second end (109);
    iii) a body region (110) with an outer diameter **d$_1$** having a first end (111) that is adjacent to the second end (109) of the shoulder region (107) and a second end (112);
    iv) a flange region (113) having at least in parts of the flange region (113) a circular shape, the flange region (113) having a first end (114) that is adjacent to the second end (112) of the body region (110) and a second end (115) that corresponds to the top end (102) of the glass syringe barrel (100), wherein the flange region (113) has an outer diameter **d$_2$** > **d$_1$** and wherein the flange region (113) is **characterized by** an outer contour **c1** at its first end (114) and an outer contour c2 at its second end (115);

wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular:

- $f_o$ and $f'_o$ are straight lines that run perpendicular to longitudinal axis $L_{barrel}$, wherein $f_o$ touches the outer contour **c1** at its deepest point $P_1$ and $f'_o$ touches the outer contour **c2** at its highest point $P_2$;
- $f(x)$ defines the absolute value of the vertical distance between any point of the outer contour **c1** and straight line $f_o$ at a position $x$ (with $f(x) = 0$ at point $P_1$), wherein x is the horizontal distance between any given point on straight line $f_o$ and point $P_0$ at which straight line $f_o$ crosses a line $L_1$ that runs parallel to longitudinal axis $L_{barrel}$ and that touches the outer surface of the body region (110) and wherein the maximum value for the term $f(x)$ in the range from x = $P_0$ to x = $P_1$ is $f(x)_{max}$ determined at position $x_{max}$, $f(x)_{max}$ representing the deepest indentation on the lower side of the flange region (113);
- wherein $f'(x)$ defines the absolute value of the vertical distance between any point of the outer contour **c2** and straight line $f'_o$ at a position $x$ (with $f'(x) = 0$ at point $P_2$);

**characterized in that** the following condition is fulfilled:

$$f'(x_{max})/f(x)_{max} > 1.$$

2. The glass syringe barrel (100) according to claim 1, wherein $f'(x_{max})$ is in the range from 0.15 to 0.7 mm.

3. The glass syringe barrel (100) according to claim 1 or 2, wherein $f(x)_{max}$ is in the range from 0.01 to 0.65 mm.

4. The glass syringe barrel (100) according to anyone of claims 1 to 3, wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular, **D1** is the distance between point $P_1$ on the outer contour **c1** and a point $P_3$ on outer contour **c2**, point $P_3$ being located vertically above point $P_1$ and $D_2$ is the distance between a point of the outer contour **c1** and outer contour **c2** at position $x_{max}$ and wherein the following condition is fulfilled:

$$D_1/D_2 < 1.6.$$

5. The glass syringe barrel (100) according to anyone of claims 1 to 4, wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular, **d'** is the distance between point $x'_{max}$ on the left side of the flange region (113) and point $x''_{max}$ on the right side of the flange region (113) on straight line $f_o$, $x'_{max}$ and $x''_{max}$ corresponding to the points at which function $f(x)$ reaches its maximum value on the left side and the right side of the flange region (113), wherein **d'** is in the range from 12.5 to 15 mm.

6. The glass syringe barrel (100) according to anyone of claims 1 to 5, wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular, **d"** is the distance between point $P'_1$ on the left side of the flange region (113) and point $P''_1$ on the right side of the flange region (113) on straight line $f_0$, wherein $d''/d_2$ is in the range from 0.8 to 0.95.

7. The glass syringe barrel (100) according to claim 6, wherein **d"** is in the range from 8 to 20 mm and $d_2$ is in the range from 12 to 22 mm.

8. The glass syringe barrel (100) according to anyone of claims 1 to 7, wherein $d_1$ is in the range from 6 to 20 mm.

9. The glass syringe barrel (100) according to anyone of claims 1 to 8, wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular, a tangent (117) that touches outer contour c2 at position $x_{max}$ includes an angle $\alpha$ with straight line $f'_o$ and wherein $\alpha$ is in the range from 10 to 28 degree.

10. The glass syringe barrel (100) according to anyone of claims 1 to 9, wherein, for any cut surface (116) of the glass syringe barrel (100) that includes the longitudinal axis $L_{barrel}$ and that is obtainable by cutting the glass syringe barrel (100) in a plane at which the shape of the flange region (113) is circular:

- $L_2$ is a straight line at touches the deepest point $P'_1$ of the outer contour **c1** on the left side of the flange region (113) and the deepest point $P''_1$ of the outer contour **c1** on the right side of the flange region (113);
- $L_3$ is a straight line at touches the outer contour **c1** on the left side of the flange region (113) at a position **x** at which **f(x)** reaches the maximum value $f(x)_{max}$ and the deepest point $P''_1$ of the outer contour **c1** on the right side of the flange region (113);

wherein straight lines $L_2$ and $L_3$ enclose an angle β of less than 3 degree.

11. The glass syringe barrel (100) according to anyone of claims 1 to 10, wherein $d_2$ - $d_1$ is in the range from 5 to 8 mm.

12. The glass syringe barrel (100) according to anyone of claims 1 to 11, wherein the flange region (113), in a plane that is perpendicular to the longitudinal axis $L_{barrel}$, has the shape of a circle or the shape of a circle where two opposite sections of the circle have been removed.

13. A process for the preparation of a glass syringe barrel (100), comprising the process steps:

I) providing a glass syringe barrel precursor (119) having a longitudinal axis $L_{bar-rel}$, the glass syringe barrel precursor (119) comprising a body region (110) with a bottom end and a top end (120);
II) heating the top end (120) of the body region (110), while rotating the glass syringe barrel precursor (119) around its longitudinal axis $L_{barrel}$, to prepare a ring of molten glass (121) at the top end (120) and forming a flange region (113) by pushing the mass of molten glass out beyond the edge of the body region (110) at the top end (120) of the glass syringe barrel precursor (119);
III) while the glass syringe barrel precursor (119) is rotating around its longitudinal axis $L_{barrel}$, shaping the flange region (113) obtained in process step II) to obtain a bottom side (114) having an outer contour c1 and a top side (115) having an outer contour **c2**, wherein the bottom side (114) of the flange region (113) is shaped by means of a shaping tool (122) that comes into contact with the bottom side (114) of the flange region (113);
wherein shaping in process step III) is performed under such conditions that a flange region (113) is obtained in which, for any cut surface (116) of the glass syringe barrel (100) that includes the longitudinal axis $L_{barrel}$ and that is obtainable by cutting the glass syringe barrel (100) in a plane at which the shape of the flange region (113) is circular,

- $f_o$ and $f'_o$ are straight lines that run vertically to longitudinal axis $L_{barrel}$, wherein $f_o$ touches the outer contour **c1** at its deepest point $P_1$ and $f'_o$ touches the outer contour **c2** at its highest point $P_2$;
- **f(x)** defines the absolute value of the vertical distance between any point of the outer contour **c1** and straight line $f_o$ at a position **x** (with **f(x)** = 0 at point $P_1$), wherein **x** is the horizontal distance between any given point on straight line $f_o$ and point $P_0$ at which straight line $f_o$ crosses a line $L_1$ that runs parallel to longitudinal axis $L_{barrel}$ and that touches the outer surface of the body region and wherein the maximum value for the term **f(x)** in the range from **x** = $P_0$ to **x** = $P_1$ is $f(x)_{max}$ determined at position $x_{max}$, $f(x)_{max}$ representing the deepest indentation on the lower side of the flange region (113);
- wherein **f'(x)** defines the absolute value of the vertical distance between any point of the outer contour **c2** and straight line $f'_o$ at a position **x** (with **f'(x)** = 0 at point $P_2$);

**characterized in that** the following condition is fulfilled:

$$f'(x_{max})/f(x)_{max} > 1.$$

14. A plurality of glass syringe barrels (100) as defined in anyone of claims 1 to 12, wherein at least one of the following conditions is fulfilled:

α) the relative standard deviation of the flange thickness **D1** is less than 6 %;
β) the relative standard deviation of the length $l_2$ of the syringe is less than 12 %.

15. A syringe comprising

A) a glass syringe barrel (100) according to anyone of claims 1 to 12 or a plurality of glass syringe barrels (100) according to claim 14;
B) a plunger stopper (103) that has been pushed into the top end (102) of the glass syringe barrel (100) or into the top end (102) of each of the glass syringe barrels (100) contained in the plurality of glass syringe barrels.

**Patentansprüche**

1. Ein Glasspritzenzylinder (100) mit einer Längsachse $L_{Zylinder}$, wobei der Glasspritzenzylinder (100) ein unteres Ende (101), durch das eine Flüssigkeit ausgestoßen werden kann, und ein oberes Ende (102), in das ein Kolbenstopfen (103) gedrückt werden kann, umfasst, wobei der Glasspritzenzylinder (100) in einer Richtung vom unteren Ende (101) zum oberen Ende (102) umfasst:

   i) einen Konusbereich (104) mit einem ersten Ende (105), das dem unteren Ende (101) des Glasspritzenzylinders (100) entspricht, und einem zweiten Ende (106);
   ii) einen Schulterbereich (107) mit einem ersten Ende (108), das an das zweite Ende (106) des Konusbereichs (104) angrenzt, und einem zweiten Ende (109);
   iii) einen Körperbereich (110) mit einem Außendurchmesser $d_1$ mit einem ersten Ende (111), das an das zweite Ende (109) des Schulterbereichs (107) angrenzt, und einem zweiten Ende (112);
   iv) einen Flanschbereich (113), der zumindest in Teilen des Flanschbereichs (113) eine kreisförmige Gestalt aufweist, wobei der Flanschbereich (113) ein erstes Ende (114), das an das zweite Ende (112) des Körperbereichs (110) angrenzt, und ein zweites Ende (115) aufweist, das dem oberen Ende (102) des Glasspritzenzylinders (100) entspricht, wobei der Flanschbereich (113) einen Außendurchmesser $d_{(2)} > d_{(1)}$ aufweist und wobei der Flanschbereich (113) durch eine Außenkontur **c1** an seinem ersten Ende (114) und eine Außenkontur c2 an seinem zweiten Ende (115) gekennzeichnet ist;

   wobei für jede Schnittfläche (116) des Glasspritzenzylinders (100), die die Längsachse $L_{Zylinder}$ enthält und durch Schneiden des Glasspritzenzylinders (100) in einer Ebene erhalten werden kann, in der die Form des Flanschbereichs (113) kreisförmig ist:

   - $f_o$ und $f'_o$ sind gerade Linien, die senkrecht zur Längsachse $L_{Barrel}$ verlaufen, wobei $f_o$ die Außenkontur **c1** des Flanschbereichs an seinem tiefsten Punkt $P_1$ berührt und $f'_o$ die Außenkontur **c2** an ihrem höchsten Punkt $P_2$ berührt;
   - $f(x)$ definiert den absoluten Wert des vertikalen Abstands zwischen einem beliebigen Punkt der Außenkontur **c1** und der Geraden $f_o$ an einer Position **x** (mit $f(x) = 0$ am Punkt $P_1$), wobei **x** der horizontale Abstand zwischen einem beliebigen Punkt auf der Geraden $f_o$ und dem Punkt $P_0$ ist, an dem die Gerade $f_o$ eine Linie $L_1$ schneidet, die parallel zur Längsachse $L_{Zylinder}$ verläuft und die Außenfläche des Körperbereichs (110) berührt, und wobei der Maximalwert für den Term $f(x)$ im Bereich von $x = P_0$ bis $x = P_1$ $f(x)_{max}$ ist, der an der Position $x_{max}$ bestimmt wird, wobei $f(x)_{max}$ die tiefste Vertiefung auf der Unterseite des Flanschbereichs (113) darstellt;
   - wobei $f'(x)$ den absoluten Wert des vertikalen Abstands zwischen einem beliebigen Punkt der Außenkontur **c2** und der Geraden $f'_o$ an einer Position x definiert (mit $f'(x) = 0$ am Punkt $P_2$);

   **dadurch gekennzeichnet, dass** die folgende Bedingung erfüllt ist:

$$f'(x_{max})/f(x)_{max} > 1.$$

2. Der Glasspritzenzylinder (100) gemäß Anspruch 1, wobei $f'(x_{max})$ im Bereich von 0,15 bis 0,7 mm liegt.

3. Glasspritzenzylinder (100) nach Anspruch 1 oder 2, wobei $f(x)_{max}$ im Bereich von 0,01 bis 0,65 mm liegt.

4. Der Glasspritzenzylinder (100) gemäß einem der Ansprüche 1 bis 3, wobei für jede Schnittfläche (116) des Glasspritzenzylinders (100), die die Längsachse $L_{Zylinder}$ aufweist und die durch Schneiden des Glasspritzenzylinders (100) in einer Ebene, in der die Form des Flanschbereichs (113) kreisförmig ist, erhalten werden kann, $D1$ der Abstand zwischen dem Punkt $P_1$ auf der Außenkontur **c1** und einem Punkt $P_3$ auf der Außenkontur **c2**, wobei der Punkt $P_3$ vertikal über dem Punkt $P_1$ liegt, und $D_2$ der Abstand zwischen einem Punkt der Außenkontur **c1** und der Außenkontur c2 an der Position $x_{max}$ ist, und wobei die folgende Bedingung erfüllt ist:

$$D_1 /D_2 < 1{,}6.$$

5. Der Glasspritzenzylinder (100) gemäß einem der Ansprüche 1 bis 4, wobei für jede Schnittfläche (116) des Glasspritzenzylinders (100), die die Längsachse $L_{Zylinder}$ und die durch Schneiden des Glasspritzenzylinders (100) in einer Ebene erhalten werden kann, in der die Form des Flanschbereichs (113) kreisförmig ist, **d'** der Abstand zwischen dem Punkt $x'_{max}$ auf der linken Seite des Flanschbereichs (113) und dem Punkt $x''_{(max)}$ auf der rechten Seite des Flanschbereichs (113) auf der Geraden $f_o$, $x'_{(max)}$ und $x''_{(max)}$ den Punkten entsprechen, an denen die Funktion **f(x)** ihren Maximalwert auf der linken Seite und der rechten Seite des Flanschbereichs (113) erreicht, wobei **d'** im Bereich von 12,5 bis 15 mm liegt.

6. Der Glasspritzenzylinder (100) gemäß einem der Ansprüche 1 bis 5, wobei für jede Schnittfläche (116) des Glasspritzenzylinders (100), die die Längsachse $L_{Zylinder}$ und die durch Schneiden des Glasspritzenzylinders (100) in einer Ebene, in der die Form des Flanschbereichs (113) kreisförmig ist, erhalten werden kann, **d''** der Abstand zwischen dem Punkt $P'_1$ auf der linken Seite des Flanschbereichs (113) und dem Punkt $P''_{(1)}$ auf der rechten Seite des Flanschbereichs (113) auf der Geraden $f_o$, wobei $d''/d_2$ im Bereich von 0,8 bis 0,95 liegt.

7. Der Glasspritzenzylinder (100) gemäß Anspruch 6, wobei d'' im Bereich von 8 bis 20 mm und $d_2$ im Bereich von 12 bis 22 mm liegt.

8. Der Glasspritzenzylinder (100) nach einem der Ansprüche 1 bis 7, wobei $d_1$ im Bereich von 6 bis 20 mm liegt.

9. Der Glasspritzenzylinder (100) gemäß einem der Ansprüche 1 bis 8, wobei für jede Schnittfläche (116) des Glasspritzenzylinders (100), die die Längsachse $L_{Zylinder}$ enthält und die durch Schneiden des Glasspritzenzylinders (100) in einer Ebene, in der die Form des Flanschbereichs (113) kreisförmig ist, erhalten werden kann, eine Tangente (117) aufweist, die die Außenkontur **c2** an der Position $x_{max}$ berührt und einen Winkel $\alpha$ mit der Geraden $f'_o$ einschließt, wobei $\alpha$ im Bereich von 10 bis 28 Grad liegt.

10. Der Glasspritzenzylinder (100) nach einem der Ansprüche 1 bis 9, wobei für jede Schnittfläche (116) des Glasspritzenzylinders (100), die die Längsachse $L_{Zylinder}$ enthält und durch Schneiden des Glasspritzenzylinders (100) in einer Ebene, in der die Form des Flanschbereichs (113) kreisförmig ist, erhältlich ist:

   - $L_2$ eine Gerade ist, die den tiefsten Punkt $P'_1$ der Außenkontur **c1** auf der linken Seite des Flanschbereichs (113) und den tiefsten Punkt $P''_1$ der Außenkontur **c1** auf der rechten Seite des Flanschbereichs (113) berührt;
   - $L_3$ ist eine Gerade, die die Außenkontur **c1** auf der linken Seite des Flanschbereichs (113) an einer Position x berührt, an der **f(x)** den Maximalwert $f(x)_{max}$ erreicht, und den tiefsten Punkt $P''_1$ der Außenkontur **c1** auf der rechten Seite des Flanschbereichs (113);

   wobei die Geraden $L_2$ und $L_3$ einen Winkel $\beta$ von weniger als 3 Grad einschließen.

11. Der Glasspritzenzylinder (100) nach einem der Ansprüche 1 bis 10, wobei $d_2$ - $d_1$ im Bereich von 5 bis 8 mm liegt.

12. Der Glasspritzenzylinder (100) nach einem der Ansprüche 1 bis 11, wobei der Flanschbereich (113) in einer Ebene, die senkrecht zur Längsachse $L_{Zylinder}$ steht, die Form eines Kreises oder die Form eines Kreises hat, bei dem zwei gegenüberliegende Abschnitte des Kreises entfernt wurden.

13. Ein Verfahren zur Herstellung eines Glasspritzenzylinders (100), umfassend die folgenden Verfahrensschritte:

   I) Bereitstellen eines Glas-Spritzenzylinder-Vorläufers (119) mit einer Längsachse $L_{Zylinder}$, wobei der Glas-Spritzenzylinder-Vorläufer (119) einen Körperbereich (110) mit einem unteren Ende und einem oberen Ende (120) umfasst;
   II) Erhitzen des oberen Endes (120) des Körperbereichs (110), während der Glas-Spritzenzylinder-Vorläufer (119) um seine Längsachse $L_{Zylinder}$, um einen Ring aus geschmolzenem Glas (121) am oberen Ende (120) herzustellen und einen Flanschbereich (113) zu bilden, indem die Masse aus geschmolzenem Glas über den Rand des Körperbereichs (110) am oberen Ende (120) des Glas-Spritzenzylinder-Vorläufers (119) hinausgedrückt wird;
   III) während sich der Glasspritzenzylinder-Vorläufer (119) um seine Längsachse $L_{Zy-linder}$ dreht, Formen des in Verfahrensschritt II) erhaltenen Flanschbereichs (113), um eine Unterseite (114) mit einer Außenkontur **c1** und eine Oberseite (115) mit einer Außenkontur **c2** zu erhalten, wobei die Unterseite (114) des Flanschbereichs (113) mittels eines Formwerkzeugs (122) geformt wird, das mit der Unterseite (114) des Flanschbereichs (113) in Kontakt kommt;
   wobei das Formen in Prozessschritt III) unter solchen Bedingungen durchgeführt wird, dass ein Flanschbereich (113) erhalten wird, in dem für jede Schnittfläche (116) des Glasspritzenzylinders (100), der die Längsachse $L_{Zylinder}$ und die durch Schneiden des Glasspritzenzylinders (100) in einer Ebene erhalten werden kann, in der die Form des Flanschbereichs (113) kreisförmig ist,

- $f_o$ und $f'_o$ gerade Linien sind, die senkrecht zur Längsachse $L_{Zylinder}$ verlaufen, wobei $f_o$ die Außenkontur **c1** an ihrem tiefsten Punkt $P_1$ berührt und $f'_o$ die Außenkontur **c2** an ihrem höchsten Punkt $P_2$ berührt;
- $f(x)$ den absoluten Wert des vertikalen Abstands zwischen einem beliebigen Punkt der Außenkontur **c1** und der Geraden $f_o$ an einer Position x definiert (mit f(x) = 0 am Punkt $P_1$), wobei **x** der horizontale Abstand zwischen einem beliebigen Punkt auf der Geraden $f_o$ und dem Punkt $P_0$ ist, an dem die Gerade $f_o$ eine Linie $L_1$ schneidet, die parallel zur Längsachse $L_{Fass}$ verläuft und die Außenfläche des Körperbereichs berührt, und wobei der Maximalwert für den Term $f(x)$ im Bereich von $x = P_0$ bis $x = P_1$ $f(x)_{max}$ ist, der an der Position $x_{max}$ bestimmt wird, wobei $f(x)_{max}$ die tiefste Vertiefung auf der Unterseite des Flanschbereichs (113) darstellt;
- wobei $f'(x)$ den absoluten Wert des vertikalen Abstands zwischen einem beliebigen Punkt der Außenkontur **c2** und der Geraden $f'_o$ an einer Position **x** definiert (mit $f'(x)$ = 0 am Punkt $P_2$);

**dadurch gekennzeichnet, dass** die folgende Bedingung erfüllt ist:

$$f'(x_{max})/f(x)_{max} > 1 .$$

14. Eine Vielzahl von Glasspritzenzylindern (100) gemäß einem der Ansprüche 1 bis 12, wobei mindestens eine der folgenden Bedingungen erfüllt ist:

α) die relative Standardabweichung der Flanschdicke **D1** weniger als 6 % beträgt;
β) die relative Standardabweichung der Länge $l_2$ der Spritze weniger als 12 % beträgt.

15. Eine Spritze, umfassend

A) einen Glasspritzenzylinder (100) gemäß einem der Ansprüche 1 bis 12 oder mehrere Glasspritzenzylinder (100) gemäß Anspruch 14;
B) einen Kolbenstopfen (103), der in das obere Ende (102) des Glasspritzenzylinders (100) oder in das obere Ende (102) jedes der in der Vielzahl von Glasspritzenzylindern enthaltenen Glasspritzenzylinder (100) gedrückt wurde.

## Revendications

1. Corps de seringue en verre (100) ayant un axe longitudinal $L_{corps}$, le corps de seringue en verre (100) comprenant une extrémité inférieure (101) à travers laquelle un liquide peut être éjecté et une extrémité supérieure (102) dans laquelle un bouchon de piston (103) peut être enfoncé, le corps de seringue en verre (100) comprenant, dans une direction allant de l'extrémité inférieure (101) à l'extrémité supérieure (102) :

i) une région conique (104) ayant une première extrémité (105) qui correspond à l'extrémité inférieure (101) du corps de seringue en verre (100) et une deuxième extrémité (106) ;
ii) une région d'épaulement (107) ayant une première extrémité (108) qui est adjacente à la deuxième extrémité (106) de la région conique (104) et une deuxième extrémité (109) ;
iii) une région de corps (110) avec un diamètre extérieur $d_1$ ayant une première extrémité (111) qui est adjacente à la deuxième extrémité (109) de la région d'épaulement (107) et une deuxième extrémité (112) ;
iv) une région de bride (113) ayant au moins dans certaines parties de la région de bride (113) une forme circulaire, la région de bride (113) ayant une première extrémité (114) adjacente à la deuxième extrémité (112) de la région de corps (110) et une deuxième extrémité (115) qui correspond à l'extrémité supérieure (102) du corps de seringue en verre (100), dans laquelle la région de bride (113) a un diamètre extérieur $d_2 > d_1$ et dans lequel la région de bride (113) est **caractérisée par** un contour extérieur **c1** à sa première extrémité (114) et un contour extérieur **c2** à sa deuxième extrémité (115) ;

dans lequel, pour toute surface de coupe (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire :

- $f_o$ et $f'_o$ sont des lignes droites perpendiculaires à l'axe longitudinal $L_{corps}$ , dans lesquelles $f_o$ touche le contour extérieur $c1$ de l' à son point le plus profond $P_1$ et $f'_o$ touche le contour extérieur $c2$ à son point le plus haut $P_2$ ;
- $f(x)$ définit la valeur absolue de la distance verticale entre tout point du contour extérieur $c1$ et la droite $f_o$ à une position $x$ (avec $f(x) = 0$ au point $P_1$ ), où x est la distance horizontale entre un point quelconque sur la ligne droite $f_o$ et le point $P_0$ où la ligne droite $f_o$ croise une ligne $L_1$ qui est parallèle à l'axe longitudinal $L_{corps}$ et qui touche la surface extérieure de la région du corps (110) et dans laquelle la valeur maximale du terme $f(x)$ dans la plage allant de $x = P_0$ à $x = P_1$ est $f(x)_{max}$ déterminée à la position $x_{max}$ , $f(x)_{max}$ représentant l'indentation la plus profonde sur le côté inférieur de la région de bride (113) ;
- où $f'(x)$ définit la valeur absolue de la distance verticale entre tout point du contour extérieur $c2$ et la ligne droite $f'_o$ à une position $x$ (avec $f'(x) = 0$ au point $P_2$ ) ;

**caractérisé en ce que** la condition suivante est remplie :

$$f'(x_{max})/f(x)_{max} > 1.$$

2. Le corps de seringue en verre (100) selon la revendication 1, dans lequel $f'(x_{max})$ est compris entre 0,15 et 0,7 mm.

3. Le corps de seringue en verre (100) selon la revendication 1 ou 2, dans lequel $f(x)_{max}$ est compris entre 0,01 et 0,65 mm.

4. Le corps de seringue en verre (100) selon l'une quelconque des revendications 1 à 3, dans lequel, pour toute surface coupée (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire, $D1$ est la distance entre le point $P_1$ sur le contour extérieur $c1$ et un point $P_3$ sur le contour extérieur $c2$, le point $P_3$ étant situé verticalement au-dessus du point $P_1$ et $D_2$ étant la distance entre un point du contour extérieur $c1$ et le contour extérieur $c2$ à la position $x_{max}$ et dans lequel la condition suivante est remplie :

$$D_1/D_2 < 1,6.$$

5. Le corps de seringue en verre (100) selon l'une quelconque des revendications 1 à 4, dans lequel, pour toute surface coupée (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire, $d'$ est la distance entre le point $x'_{max}$ sur le côté gauche de la région de bride (113) et le point $x''_{(max)}$ sur le côté droit de la région de bride (113) sur la ligne droite $f_o$, $x'_{(max)}$ et $x''_{(max)}$ correspondant aux points auxquels la fonction f(x) atteint sa valeur maximale sur le côté gauche et le côté droit de la région de bride (113), où $d'$ est compris entre 12,5 et 15 mm.

6. Le corps de seringue en verre (100) selon l'une quelconque des revendications 1 à 5, dans lequel, pour toute surface de coupe (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire, $d''$ est la distance entre le point $P'_1$ situé sur le côté gauche de la région de bride (113) et le point $P''_{(1)}$ sur le côté droit de la région de bride (113) sur la ligne droite f(0) ,où $d''/d(2)$ est compris entre 0,8 et 0,95.

7. Le corps de seringue en verre (100) selon la revendication 6, dans lequel d" est compris entre 8 et 20 mm et $d_2$ est compris entre 12 et 22 mm.

8. Le corps de seringue en verre (100) selon l'une quelconque des revendications 1 à 7, dans lequel $d_1$ est compris entre 6 et 20 mm.

9. Le corps de seringue en verre (100) selon l'une quelconque des revendications 1 à 8, dans lequel, pour toute surface coupée (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire, une tangente (117) qui touche le contour extérieur $c2$ à la position $x_{max}$ comprend un angle $\alpha$ avec la ligne droite $f'_o$ et dans lequel $\alpha$ est compris entre 10 et 28 degrés.

10. Le corps de seringue en verre (100) selon l'une quelconque des revendications 1 à 9, dans lequel, pour toute surface coupée (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire :

- $L_2$ est une ligne droite qui touche le point le plus profond $P'_1$ du contour extérieur **c1** sur le côté gauche de la région de bride (113) et le point le plus profond $P''_1$ du contour extérieur **c1** sur le côté droit de la région de bride (113) ;
- $L_3$ est une ligne droite qui touche le contour extérieur **c1** sur le côté gauche de la zone de bride (113) à une position **x** à laquelle **f(x)** atteint la valeur maximale $f(x)_{max}$ et le point le plus profond $P''_1$ du contour extérieur **c1** sur le côté droit de la zone de bride (113) ;

dans lequel les lignes droites $L_2$ et $L_3$ forment un angle $\beta$ inférieur à 3 degrés.

11. Le corps de seringue en verre (100) selon l'une quelconque des revendications 1 à 10, dans lequel $d_2 - d_1$ est compris entre 5 et 8 mm.

12. Le corps de seringue en verre (100) selon l'une quelconque des revendications 1 à 11, dans lequel la région de bride (113), dans un plan perpendiculaire à l'axe longitudinal $L_{corps}$, a la forme d'un cercle ou la forme d'un cercle dont deux sections opposées ont été retirées.

13. Un procédé de fabrication d'un corps de seringue en verre (100), comprenant les étapes suivantes :

    I) fournir un précurseur de corps de seringue en verre (119) ayant un axe longitudinal $L_{corps}$, le précurseur de corps de seringue en verre (119) comprenant une région de corps (110) avec une extrémité inférieure et une extrémité supérieure (120) ;
    II) chauffer l'extrémité supérieure (120) de la région de corps (110), tout en faisant tourner le précurseur de corps de seringue en verre (119) autour de son axe longitudinal $L_{corps}$, afin de préparer un anneau de verre fondu (121) à l'extrémité supérieure (120) et de former une région de bride (113) en poussant la masse de verre fondu au-delà du bord de la région de corps (110) à l'extrémité supérieure (120) du précurseur de corps de seringue en verre (119) ;
    III) tandis que le précurseur de corps de seringue en verre (119) tourne autour de son axe longitudinal $L_{corps}$, façonner la région de bride (113) obtenue à l'étape II) du procédé pour obtenir une face inférieure (114) ayant un contour extérieur **c1** et une face supérieure (115) ayant un contour extérieur **c2**, dans lequel la face inférieure (114) de la région de bride (113) est formée au moyen d'un outil de formage (122) qui entre en contact avec la face inférieure (114) de la zone de bride (113) ;
    dans lequel le façonnage de l'étape III) est réalisé dans des conditions telles qu'on obtient une région de bride (113) dans laquelle, pour toute surface coupée (116) du corps de seringue en verre (100) qui comprend l'axe longitudinal $L_{corps}$ et qui peut être obtenue en coupant le corps de seringue en verre (100) dans un plan dans lequel la forme de la région de bride (113) est circulaire,

        - $f_o$ et $f'_o$ sont des lignes droites qui s'étendent verticalement par rapport à l'axe longitudinal $L_{corps}$, dans lequel $f_o$ touche le contour extérieur **c1** en son point le plus profond $P_1$ et $f'_o$ touche le contour extérieur **c2** en son point le plus haut $P_2$ ;
        - **f(x)** définit la valeur absolue de la distance verticale entre tout point du contour extérieur **c1** et la ligne droite $f_o$ à une position **x** (avec **f(x)** = 0 au point $P_1$), où **x** est la distance horizontale entre tout point donné sur la ligne droite $f_o$ et le point $P_0$ où la ligne droite $f_o$ croise une ligne $L_1$ qui est parallèle à l'axe longitudinal $L_{corps}$ et qui touche la surface extérieure de la région du corps, et dans laquelle la valeur maximale du terme **f(x)** dans l'intervalle de $x = P_0$ à $x = P_1$ est $f(x)_{max}$ déterminée à la position $x_{max}$, $f(x)_{max}$ représentant l'indentation la plus profonde sur le côté inférieur de la région de bride (113) ;
        - où **f'(x)** définit la valeur absolue de la distance verticale entre tout point du contour extérieur **c2** et la ligne droite $f'_o$ à une position **x** (avec **f'(x)** = 0 au point $P_2$) ;

    **caractérisé en ce que** la condition suivante est remplie :

$$f'(x_{max}))/f(x)_{max} > 1 \ .$$

14. Une pluralité de corps de seringues en verre (100) tels que définis dans l'une quelconque des revendications 1 à 12, dans lesquels au moins l'une des conditions suivantes est remplie :

    $\alpha$) l'écart type relatif de l'épaisseur de bride **D1** est inférieur à 6 % ;
    $\beta$) l'écart type relatif de la longueur $l_2$ de la seringue est inférieur à 12 %.

15. Une seringue comprenant

A) un corps de seringue en verre (100) selon l'une quelconque des revendications 1 à 12 ou une pluralité de corpss de seringue en verre (100) selon la revendication 14 ;
B) un bouchon de piston (103) qui a été enfoncé dans l'extrémité supérieure (102) du corps de seringue en verre (100) ou dans l'extrémité supérieure (102) de chacun des corpss de seringue en verre (100) contenus dans la pluralité de corpss de seringue en verre.

# Fig. 1

# Fig. 2

# Fig. 3

Fig. 4

# Fig. 5

## Fig. 6

100

$L_{barrel}$

A

116

113

116

$L_{barrel}$

B

# Fig. 7

$\mathbf{L_{barrel}}$

113

A

$\mathbf{L_{barrel}}$

116a

113

B

116a     116b     116a

# Fig. 8

# Fig. 9

123

120 / 115

121

$\gamma$

114

122

110

119

$L_{barrel}$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018326156 A1
- US 2019125978 A1
- US 2018296762 A1
- US 2003141268 A1
- US 2021085877 A1

**Non-patent literature cited in the description**

- European Pharmacopoeia. 2011